# EUROPEAN PATENT SPECIFICATION

(11) **EP 3 013 366 B1**
(45) Date of publication and mention of the grant of the patent: **25.08.2021**
(21) Application number: 14739078.5
(22) Date of filing: 27.06.2014
(51) Int. Cl.: A61K 38/57, A61K 39/395, A61K 31/4365, A61K 31/519, A61K 31/60, A61K 31/616, A61K 31/727, A61K 45/06, C07K 16/36, A61P 3/00, A61P 7/02, A61P 7/10, A61P 9/00, A61P 9/10, A61P 11/00, A61P 21/00, A61P 25/00, A61P 29/00, A61P 43/00

(54) **COMBINATION THERAPY USING A FACTOR XII INHIBITOR AND A C1-INHIBITOR**
KOMBINATIONSTHERAPIE MIT EINEM FAKTOR XII-HEMMER UND EINEM C1-HEMMER
THÉRAPIE COMBINÉE UTILISANT UN INHIBITEUR DU FACTEUR XII ET L'INHIBITEUR C1

(30) Priority: 28.06.2013 US 201361840662 P; 16.07.2013 EP 13176605
(43) Date of publication of application: 04.05.2016
(73) Proprietor: CSL Behring GmbH, 35041 Marburg (DE)
(72) Inventor: NOLTE, Marc, 35041 Marburg (DE); PRAGST, Ingo, 34549 Edertal (DE)
(74) Representative: Binsack, Beate
(86) International application number: PCT/EP2014/063691
(87) International publication number: WO 2014/207199

(56) References cited:
- EP-A1- 2 497 489
- EP-A2- 0 952 215
- WO-A1-99/36439
- WO-A1-2008/098720
- WO-A2-2008/091692
- JP-A- H11 209 399
- US-A- 6 090 777
- US-A1- 2007 196 367
- US-A1- 2008 254 039
- US-A1- 2012 088 728
- US-A1- 2012 189 626
- None

## Description

This disclosure relates to the use of at least one C1-Inhibitor (C1 esterase inhibitor or C1-INH) and at least one Factor XII inhibitor in the treatment of disorders of the contact activation system. Compositions comprising at least one C1-INH and at least one Factor XII inhibitor are also provided.

Two converging pathways for coagulation exist that are triggered by either "extrinsic" (vessel wall) or "intrinsic" (blood-borne) components of the vascular system. The "intrinsic" or contact activation pathway is initiated when Factor XII (FXII, Hageman factor) comes into contact with negatively charged surfaces in a reaction involving high molecular weight kininogen and plasma kallikrein. Factor XII is a serine protease and, once activated (FXIIa), it further activates circulating FXII in a positive feedback reaction (directly or via activation of prekallikrein). FXIIa also activates Factor XI and blood coagulation proceeds in a reaction cascade involving the activation of further factors by limited proteolysis culminating in the generation of thrombin, which converts plasma fibrinogen to fibrin and activates platelets.

Although FXII is the initiating factor in the intrinsic pathway, FXII deficiency is not associated with an increase in spontaneous or injury-related bleeding complications (Ratnoff & Colopy, J Clin Invest 34, 602-13 (1955)). Indeed, humans that are deficient in FXII do not suffer from abnormal bleeding even during major surgical procedures (Colman, R. W. Hemostasis and Thrombosis. Basic Principles & Clinical Practice (eds. Colman et al.) 103-122 (Lippincott Williams & Wilkins, Philadelphia, 2001)).

In some prior reports, FXII deficiency had been associated with increased risk of venous thrombosis. (Kuhli et al., Am. J. Opthalmol. 137, 459-464 (2004): Halbmayer et al., Wiener Med. Wochschr. 143, 43-50 (1993).) Studies and case reports supporting this idea referred to the index case for FXII deficiency, John Hageman, who died of pulmonary embolism. However, this hypothesis was challenged by a recent reevaluation of several case reports linking FXII deficiency with thrombosis. (Girolami et al., J. Thromb. Thrombolysis 17, 139-143 (2004)). In most cases the authors identified concomitant congenital or acquired prothrombotic risk factors in combination with FXII deficiency that could be responsible for the thrombotic event independently of FXII. Large epidemiological studies using well characterized patients (Koster et al., Br. J. Haematol. 87, 422-424 (1994)) and FXII-deficient families (Zeerleder et al., Thromb. Haemost. 82, 1240-1246 (1999)) indicate that there is no correlation between FXII deficiency and a pro-thrombotic risk.

More recently, Kleinschnitz et al. reported a role for FXII in pathological thrombosis in cerebral ischemia. (Kleinschnitz et al., J. Exp. Med. 203:513-8 (2006)). And studies in FXII-deficient mice have suggested that FXII is involved in pathological arterial thrombus formation by stabilizing newly generated thrombi. See US 2008/0254039 and WO 2006/066878 which disclose the use of a FXII inhibitor for preventing the formation and/or the stabilization of thrombi but disclose neither any actual data nor any combined administration of a FXII inhibitor with a C1-inhibitor.

WO 2008/098720 discloses the use of particular FXIIa inhibitors, i.e. mutant Kazal inhibitors derived from SPINK-1 which are mutated in order to increase homology to Infestin-4, for the treatment of diseases related to arterial thrombus formation but fails to disclose any combination therapy.

C1-Inhibitor (C1-INH) is a serine protease inhibitor and the physiological inhibitor of the endogenous contact activation system. It inhibits several proteases associated with the complement, kallikrein-kinin, and coagulation systems, including FXII. Based on its central role in regulating the classical complement pathway and the involvement of complement-mediated inflammation in reperfusion injury, the effects of C1-INH administration on the neurological deficit and infarction size following stroke have been investigated. It was found that C1-INH has potent neuroprotective properties in mouse models of cerebral ischemia. (See, e.g., De Simoni et al., J. Cereb. Blood Flow Metab. 23(2): 232-239 (2003)). Furthermore, C1-INH is also an approved therapy for patients with hereditary angioedema (HAE).

Thrombus (blood clot) formation is a physiological response to injury that reduces blood loss. Vessel wall injury triggers sudden adhesion and aggregation of blood platelets, followed by the activation of the plasma coagulation system and the formation of fibrin-containing thrombi, which seal the site of injury (i.e. the injury in the vessel wall). These events limit posttraumatic blood loss but, under pathological conditions, may also occlude diseased vessels, leading to ischemia and/or infarction of vital tissues and organs. Subsequently, inflammation and/or other physiological responses secondary to occlusion or disruption of blood flow can further damage a tissue or organ. Other diseases and disorders associated with the contact activation pathway, such as those of the kinin-kallikrein or the complement systems, can likewise lead to damage of vital organs and tissues.

Despite these initial studies, there remains a need for improved treatment of disorders of the contact activation system, such as thrombosis, and particularly for treatments that can provide improved efficacy and/or enable the use of lower doses of costly therapeutic agents. In that regard, the inventors have discovered that treatment of and protection from disorders of the contact activation system, such as arterial thrombosis, could be synergistically enhanced by administering at least one FXII inhibitor (e.g., rHA-Infestin-4) and at least one C1-INH (e.g., BERINERT®).

Accordingly, disclosed herein are combination therapies comprising the use of at least one C1-Inhibitor (C1 esterase inhibitor or C1-INH) and at least one Factor XII inhibitor (FXII inhibitor), wherein the FXII inhibitor is not C1-INH, in the treatment of disorders of the contact activation system. The disorder of the contact activation system can be selected from a thrombotic disorder, and a disorder related to kinin formation wherein the disorder related to kinin formation is selected from hereditary angioedema (HAE), secondary brain edema, edema of the central nervous system, hypotensive shock, and edema during or after contacting blood with an artificial surface, and therapy can comprise administering an effective amount of at least one FXII inhibitor and at least one C1-Inhibitor C1-INH wherein the at least one C1-INH is a plasma-derived human C1 Esterase Inhibitor and/or a recombinant human C1 Esterase Inhibitor.

In some embodiments, the at least one FXII inhibitor can comprise a wild type Infestin-4 polypeptide sequence (SEQ ID NO: 1) or an Infestin-4 sequence harboring 1-5 amino acid mutations outside of N-terminal amino acid positions 2-13 of SEQ ID NO: 1 and/or a homology of at least 70%, 80%, 85%, 90%, 95%, 98%, or 99% to SEQ ID NO: 1 and retaining six conserved cysteine residues from SEQ ID NO: 1. In certain embodiments, the at least one FXII inhibitor can comprise an anti-FXII antibody wherein the anti-FXII antibody binds to and inhibits FXIIa activation and/or activity. In some embodiments, the at least one FXII inhibitor can be linked to a fusion partner (e.g., a half-life enhancing polypeptide) either directly or via a linker.

The at least one C1-INH is a plasma-derived human C1 Esterase Inhibitor and/or a recombinant human C1 Esterase Inhibitor.

In various embodiments, the at least one FXII inhibitor and the at least one C1-INH are administered prophylactically, or they are administered after a patient develops a disorder of the contact activation system. The at least one FXII inhibitor and the at least one C1-INH can be administered at the same time, the FXII inhibitor can be administered first, or the C1-INH can be administered first. The at least one FXII inhibitor and the at least one C1-INH can be administered once, or multiple times (e.g., repeated administration to treat a chronic disorder, as a repeated prophylactic treatment, or as a repeated treatment during or following an incidence of a disorder of the contact activation system).

### BRIEF DESCRIPTION OF THE DRAWINGS

Figure 1 shows the time course of arterial occlusion, as well as the occlusion rates, in an FeCl₃-induced arterial thrombosis model in rats following treatment with a Factor XII inhibitor (rHA-Infestin-4), C1-INH, or a combination of the two.
Figure 2 shows the occlusion rate in an FeCl₃-induced arterial thrombosis model in rats following treatment with a Factor XII inhibitor (rHA-Infestin-4) or C1-INH individually or in combination.

### DETAILED DESCRIPTION OF CERTAIN EMBODIMENTS

The embodiments of the disclosure pertain to methods comprising administering at least one Factor XII (FXII) inhibitor and at least one C1 esterase inhibitor (C1-INH) to a patient to treat disorders of the contact activation system. In some embodiments, the combination provides synergistic effects as compared to either component when administered as a monotherapy. In some embodiments, the synergistic effectiveness of the combination enables the use of reduced dosages for either or both components of the combination, potentially reducing treatment cost and the risk of potential side effects such as an immune response to either or both components. In addition, a combination therapy can interact with multiple pathways underlying the pathophysiology of the diseases treated by the combination, potentially providing more effective treatment to a broader range of patient populations.

### Definitions

In this disclosure, the use of the singular (such as "a" or "the") includes the plural unless specifically stated otherwise. Also in this disclosure, the use of "or" means "and/or" unless stated otherwise. Furthermore, the use of the term "including," as well as other forms, such as "includes" and "included," are not limiting. Any range described here will be understood to include the endpoints and all values between the endpoints.

The section headings are for organizational purposes only and are not to be construed as limiting the subject matter described.

As used herein, a "FXII inhibitor" refers to an inhibitor of either or both of Factor XII (prior to activation) and activated Factor XII (FXIIa), while the terms "C1 Inhibitor," "C1 esterase inhibitor," and "C1-INH" refer to serine protease inhibitors that bind to and inhibit proteins in the C1 complex, preventing activation of circulating proteases and/or inhibiting activated proteases associated with the complement system, the kallikrein-kinin system, and/or the coagulation system. A C1-INH can be plasmatic (i.e. plasma-derived), recombinant, or a combination of the two. A C1-INH can be identical to the naturally occurring human protein or a functional variant thereof. FXII inhibitors and C1-INHs encompass functional variants and fragments of the wild-type inhibitors. A functional variant or fragment is a molecule that retains at least 50% (e.g., 50, 60, 70, 80, 90, 95, 99, or 100%, or any percentage in between) of the ability of the wild-type molecule to inhibit FXII or C1 activity.

A "disorder of the contact activation system," as used herein, refers to a disorder that pathophysiologically involves, affects, and/or alters the activity levels of one or more of the coagulation system, the complement system and the kallikrein-kinin system, all of which are involved with or originate in the contact activation pathway. Disorders of the contact activation system include thrombotic disorders, disorders related to kinin formation, interstitial lung disorders, disorders of the complement activation system, neurological inflammatory disorders, or ischemia-reperfusion injuries (IRI), all of which are associated with pathologic activity of the contact activation pathway.

As used here, the terms "treat" and "treating" encompass preventing, inhibiting, eliminating, delaying the onset, slowing, lessening, reducing the severity, or ameliorating at least one sign, symptom, or aspect of a disorder or disease. Treating does not require a complete elimination of symptoms, in that it encompasses but is not equivalent to "cure" or "curing". In some embodiments, a patient can be treated to prevent a disorder, meaning either administering therapy to a subject known to be at risk for developing a disorder of the contact activation system (for example, the subject has one or more predisposing factor for one or more of the disorders described above) or, in those disorders where recurrence is likely, administering therapy after a primary event, e.g., inhibiting a second event in a patient who has experienced a primary event. For example, treating a thrombotic disorder can include preventing the formation, growth, or embolism of a thrombus. In some embodiments, "treat" or "treating" can also include ameliorating the effects of a disorder or disease. The terms "ameliorating" and "ameliorating the effects" mean that some aspect of the disorder or disease that produces an impairment of one or more patient function is improved. For example, treating a stroke can include preventing a stroke, ameliorating the effects of the stroke, or reducing the severity of the stroke (as measured, e.g., by the extent of tissue damage or the loss of neurological function).

As used herein, a "patient" is any human or animal that has, has had, or is likely to develop a disorder of the contact activation pathway and who could benefit from the administration of at least one FXII inhibitor and at least one C1-INH. The administration of a FXII inhibitor and/or a C1-INH can be by any known method of delivering a pharmaceutical or therapeutic agent to a patient, including, without limitation, parenteral administration (e.g., subdural, subcutaneous, intravenous, intra-arterial, intramuscular, intrathecal, intranasal, intratracheal, inhalative, and/or intraperitoneal injection), oral, and/or rectal administration, as well as administration by instillation, spray application, and/or infusion techniques. In certain embodiments, the administration can be done intravenously or subcutaneously.

As used herein, an "antibody" includes any polypeptide comprising a functional antigen-binding site, including immunoglobulins and antigen-binding parts or fragments thereof. A functional antigen-binding part or fragment is a molecule that retains at least 50% (e.g., 50, 60, 70, 80, 90, 95, 99, or 100%, or any percentage in between) of the ability of the full-length antibody to bind to and inhibit the antigen. The term antibody includes but is not limited to polyclonal, monoclonal, monospecific, polyspecific, non-specific, humanized, fully human, camelized, single-chain, chimeric, synthetic, recombinant, hybrid, mutated, back-mutated, and CDR-grafted antibodies. The term also includes antibody fragments such as Fab, F(ab')2, Fv, scFv, Fd, dAb, VHH (also referred to as nanobodies), and other antibody fragments or variants that retain antigen-binding function, including bi-specific or multi-specific antibodies. An antibody can be of any isotype, including IgA, IgD, IgE, IgG, and IgM. As used herein, an "antigen" is a target molecule that is capable of being bound by an antibody. As used herein, the term "antigen-binding site" refers to the part of an antibody molecule capable binding to or complementary to a part or all of an antigen.
As used herein, a "synergistic" effect refers to a therapeutic effect observed after administering at least one C1-INH and at least one FXII inhibitor that is greater than the sum of the therapeutic effects observed from administering a C1-INH as a monotherapy and a FXII inhibitor as a monotherapy. For example, a synergistic effect could include a greater increase in blood flow and/or reduction in thrombosis incidence after administering at least one C1-INH and at least one FXII inhibitor to a patient at risk for or suffering from a thrombotic event than that observed from the sum of the monotherapies. Likewise, another example of a synergistic effect in a patient being treated for a disorder of the contact activation system could include a greater reduction in the amount of tissue damage or the level of functional loss (e.g., the loss of neurological function) beyond that expected from adding the effects of a C1-INH and a FXII inhibitor monotherapy.

### I. Disorders of the Contact Activation System

In some embodiments, at least one FXII inhibitor and at least one C1-INH are disclosed and can be used in methods of treating a disorder of the contact activation system. The methods can comprise administering to a subject in need thereof at least one FXII inhibitor and at least one C1-INH. Accordingly, the invention also provides one or more pharmaceutical compositions comprising at least one FXII inhibitor and/or at least one C1-INH in pharmaceutically acceptable excipients or carriers for use in treating disorders of the contact activation system. Similarly, the invention also provides the use of one or more compositions comprising at least one FXII inhibitor and/or at least one C1-INH in the preparation of a medicament for treating a disorder of the contact activation system. The at least one FXII inhibitor and at least one C1-INH can be used alone or additional therapeutic compounds can also be administered.

A "thrombotic disorder" treated using the methods and compositions disclosed herein is any disorder/disease characterized by the formation of a thrombus (blood clot) that obstructs or decreases blood flow. The thrombus may remain local to where it formed, or it may detach to occlude blood flow downstream (thromboembolism). In some embodiments, a thrombosis may occur in a vein (venous thrombosis) or in an artery (arterial thrombosis) anywhere in the body, including the heart and brain. When the thrombosis occurs in the coronary circulation, it is referred to as a coronary thrombosis. When the thrombosis occurs in the cerebral circulation, it is referred to as a cerebral thrombosis.

A thrombotic disorder can include a venous, arterial, or capillary thrombus, thrombus formation in the heart, chronic and/or acute thromboembolism (e.g. pulmonary embolism, cerebral thromboembolism following atrial fibrillation-induced thrombus formation (e.g., stroke prevention in atrial fibrillation (SPAF)), thrombus formation as a result of contacting the blood of a human or animal subject with an artificial surface (e.g. in patients with valve replacements, stents, percutaneous coronary intervention (PCI), extracorporeal membrane oxygenation (ECMO), or undergoing cardiopulmonary bypass surgery (CPB surgery)). The thrombus can cause a stroke, myocardial infarction, deep vein thrombosis (DVT), portal vein thrombosis, thromboembolism, renal vein thrombosis, jugular vein thrombosis, cerebral venous sinus thrombosis, Budd-Chiari syndrome, Paget-Schroetter disease, or silent brain ischemia (SBI).

A disorder related to kinin formation treated using the methods and compositions disclosed herein can include hereditary angioedema (HAE), secondary brain edema, edema of the central nervous system, hypotensive shock, disseminated intravascular coagulation (DIC), or sepsis.

Once a thrombus forms, blood flow in the vessel can be reduced or blocked, causing ischemia of the surrounding tissue or the respective organ. If blood flow is not restored, the tissue associated with the blood vessel can be damaged or destroyed (referred to as an "infarct"). Depending upon the location of the thrombus, the thrombotic disorder is known by various names. Accordingly, in various embodiments, methods of treating the formation of a thrombus or a condition caused by a thrombus at a particular site are disclosed, as well as compositions comprising at least one FXII inhibitor and at least one C1-INH and their use in those methods.

Stroke is one example of a condition that can be caused by a thrombus or the formation/deposition of thrombus/thromboembolism at a particular site and which can, in some embodiments, be treated using the compositions and combinations disclosed herein.

Stroke (also referred to as a cerebrovascular accident (CVA) or cerebral infarction) involves a decline in brain function due to lack of blood flow, irrespective of the cause. When the stroke is caused by a lack of blood flow to a part of the brain (e.g., due to a thrombus blocking the blood flow), it is referred to as an ischemic stroke. There are three main causative reasons for ischemic stroke: thrombosis (obstruction of a blood vessel by a blood clot forming locally), embolism (obstruction of a blood vessel by a blood clot forming elsewhere in the body), and systemic hypoperfusion (general decrease in blood supply, e.g. in shock). Thrombosis and/or embolism can occur in arteries, veins, arterioles, venules, and/or capillaries. The term "ischemic stroke" includes stroke caused by a locally-formed thrombus (i.e., "thrombotic stroke") in any area of the brain, whether a large vessel (e.g., internal carotid artery, vertebral vessel, circle of Willis), small vessel, capillary, or sinus (e.g., cerebral venous sinus thrombosis), as well as stroke caused when a blood clot that has formed elsewhere inside or outside the brain embolizes and then lodges in a blood vessel in the brain ("embolic stroke"). Ischemic stroke also includes transient ischemic attack (also known as "mini stroke"). In transient ischemic attack, the neurological dysfunction is transient due to an ischemic event, but, in contrast to other types of stroke, does not include tissue damage (infarction) and the symptoms are reversible. In some embodiments, the present disclosure provides methods and compositions for treating ischemic stroke.

Stroke can also be caused by haemorrhagic processes, i.e., hemorrhagic stroke. Hemorrhage (e.g., an intracranial hemorrhage) is generally due to a rupture of a blood vessel or an abnormal vascular structure anywhere within the skull vault, both scenarios ultimately leading to a damage of brain tissue. Some hemorrhages can develop inside areas of ischemia ("hemorrhagic transformation"). A hemorrhagic stroke can be an intra-axial hemorrhage (blood inside the brain) or an extra-axial hemorrhage (blood inside the skull but outside the brain). In some embodiments, the methods and compositions disclosed herein can be used to treat hemorrhagic stroke.

Following a stroke, reperfusion can result in activation of inflammatory and/or other cascades that can further exacerbate the extent of tissue damage. Reperfusion and inflammation can activate various cascades, including those in the coagulation, kallikrein-kinin, and complement pathways. Accordingly, in some embodiments, the methods and compositions disclosed herein can be used to treat these secondary aspects of stroke (e.g., the secondary aspects of ischemic or hemorrhagic stroke).

Myocardial infarction is another condition that can be caused by a thrombus. A myocardial infarct refers generally to the death of heart tissue. A myocardial infarction can be caused, e.g., by a thrombus in the coronary circulation, often in the coronary artery. Accordingly, in one embodiment the invention provides methods and compositions for use in treating myocardial infarction caused by a thrombus.

Other thrombotic conditions of various tissues/sites include deep vein thrombosis (DVT), portal vein thrombosis, renal vein thrombosis, jugular vein thrombosis, Budd-Chiari syndrome (blockage of the hepatic vein or inferior vena cava), Paget-Schroetter disease (obstruction of an upper extremity vein), hepatic artery thrombosis (a complication of liver transplantation), and other vascular thrombotic events in patients at risk for such events (e.g., post-orthopedic surgery, cancer patients, surgical patients, immobilized patients). Accordingly, disclosed in these embodiments are methods and compositions for use in treating a thrombotic condition of one or more of these tissues/sites.

If a thrombus breaks free (i.e., embolizes), it may block blood flow in any of the tissue/organ sites noted above. A common site of thromboembolism is the lungs (termed "pulmonary embolism"). Arterial emboli also occlude vessels in the limbs. Thus, in some embodiments, methods and compositions are disclosed for use in treating a thromboembolism, such as a pulmonary or arterial embolism.

Multiple small thrombi may also form throughout blood vessels in conditions involving excessive consumption of coagulation factors. This condition, known as disseminated intravascular coagulation (DIC), leads to hemorrhaging and ischemic necrosis of multiple tissues. Thus, in some embodiments, methods and compositions are disclosed for use in treating a disseminated intravascular coagulation and compositions for use in treating a disseminated intravascular coagulation.

Any of a number of conditions can cause or contribute to formation of a thrombus. For example, thrombosis can be caused by an abnormality in the composition of the blood (e.g., hypercoagulability or thrombophilia), the quality of the vessel wall (endothelial cell injury or atherosclerosis), and/or the nature of the blood flow (stasis, turbulence).

Examples of predisposing factors for thrombus formation include atherosclerosis, the presence of cardiovascular prosthetic devices (e.g., stents or valve replacements), the presence of cardiac valve disease, medical procedures where blood transiently comes into contact with foreign surfaces (e.g. CPB, ECMO, BVAD, percutaneous coronary intervention), atrial fibrillation (which can cause stagnant blood flow in the left atrium or left atrial appendage), cancer, antiphospholipid antibodies, heparin-induced thrombocytopenia, infection, use of oral contraceptives, prolonged immobilization (e.g., due to surgery, obesity, heart failure, paralysis, long-haul flights, or pregnancy), tissue injury (e.g., due to trauma or surgery), and/or genetic defects (e.g., Factor V Leiden mutation, prothrombin 20210 gene mutation, deficiencies in protein C, protein S, protein Z, or antithrombin). Thrombosis may follow catheter insertion in procedures such as percutaneous coronary intervention. Other predisposing factors include a family history of thrombosis, one or more prior episodes of thrombosis, a thrombosis before the age of 50, or thrombosis at an atypical site.

Clinical assays are available to detect defects, such as Factor V resistance to activated protein C (APC) and the various protein deficiencies. The presence of a thrombus can be confirmed, e.g., using angiography, ultrasonography, MRI, or a CT scan. In some assays, a thrombus is detected, whereas in other assays an area of infarct indicates whether a thrombus is or was present.

Other conditions suitable for treatment by the methods and compositions disclosed herein include administration intended to prevent the formation and/or reduce the size of a secondary edema of the central nervous system. Secondary edema can comprise an increase in brain or spinal cord volume resulting from localized or abnormal accumulation of fluid, e.g., accumulation within the brain parenchyma. Secondary edema is a common cause of secondary infarct growth and subsequent neurological damage. For example, the methods and compositions disclosed herein can be used to treat a patient who is at risk of developing or has developed secondary edema of the central nervous system due to a brain or spinal cord injury, or due to diseases such as stroke.

In some embodiments, a treatment prevents the formation or limits/inhibits the growth in the size of a thrombus. In one embodiment, a treatment limits the ultimate size (e.g., volume) of an infarct. In an embodiment, a treatment prevents or limits the extent of reperfusion injury following an infarct. In some embodiments, a treatment is administered in combination with one or more additional agents that lyse or otherwise remove thrombi (e.g. tPA, Plasmin) while the combination therapy disclosed herein prevents reformation or growth of thrombi.

In an embodiment, treatment results in an improved and/or restored function in a tissue to which blood flow has been diminished or blocked by the thrombus. In one embodiment, the treatment results in an improvement in blood flow at the site of the thrombus as measured, for example, by ultrasound doppler, laser doppler flowmetry, MRI (e.g., measuring wash-in, wash-out kinetics of tracers), strain gage plethysmography, impedance plethysmography, photoelectric plethysmography, thermal convection probes, and/or other suitable techniques for measuring blood flow. In an embodiment, the treatment reduces or prevents neurological damage following a stroke, as measured using standard clinical neurological or psychological measures.

The at least one FXII inhibitor and the at least one C1-INH can be administered together or separately. In one embodiment, C1-INH and FXII inhibitor are administered together in a single intravenous administration. In another embodiment, the FXII inhibitor is administered first, followed by administration of the C1-INH. In certain embodiments, the C1-INH is administered directly after, or approximately 5, 10, 20, 30, 40, 50, 60, 90, 120, 150, 180, or more minutes after administration of the FXII inhibitor (or at any time point in between). In other embodiments, the C1-INH is administered first, followed by administration of the FXII inhibitor. In certain embodiments, the FXII inhibitor is administered directly after, or approximately 5, 10, 20, 30, 40, 50, 60, 90, 120, 150, 180, or more minutes after administration of the C1 INH (or at any time point in between).

Treatment with at least one C1-INH and at least one FXII inhibitor can be done prophylactically to prevent a disorder of the contact activation system, e.g., to prevent a thrombotic event. Treatment can also be administered immediately or at some time point after an episode of a disorder of the contact activation system. For example, treatment can be administered directly after the start of reperfusion following a thrombotic or IRI disorder (e.g., to prevent secondary ischemia or inflammation damage), or up to about 48 hours, or up to about 240 hours, following an episode (i.e., an injury) of a disorder of the contact activation system, such as the formation of a thrombus. Thrombus formation and other episodes of disorders can be detected, e.g., by clinical signs/symptoms and/or by the results of diagnostic imaging or in vitro assays.

In certain embodiments, treatment is begun immediately after, or less than about 6 hours after an acute episode of a disorder of the contact activation system (e.g., less than about 6 hours after thrombus formation, stroke, or reperfusion in an IRI disorder). In some embodiments, treatment is begun up to about 48, or up to about 240 hours later. In some embodiments, treatment is administered immediately after, or about 5, 10, 20, 30, 40, or 50 minutes, or 1, 2, 3, 4, 5, 6, 7, 8, 9, 10, 11, 12, 13, 14, 15, 16, 17, 18, 19, 20, 21, 22, 23, 24, 36, 48, 72, 96, 120, or 240 hours following the formation of a thrombus or other acute episode of a disorder of the contact activation system (or at any time point in between). Treatment even after 6 hours, and even after about 48 or 240 hours, may have benefits. In some embodiments, treatment is administered as soon as possible after the occurrence of the initial injury, and preferably less than 1 hour, less than 6 hours, or less than 48 hours following injury. In some embodiments, treatment occurs no later than about 240 hours following initial injury, or no more than about 48 hours following injury, or no more than about 6 hours following injury, or no more than about 1 hour following injury.

In some embodiments, administration of at least one C1-INH and at least one FXII inhibitor can be done immediately or up to about 10 days after the start of reperfusion following an initial injury (e.g., following a thrombotic occlusion). In some embodiments, treatment is administered as soon as possible after the start of reperfusion, and preferably less than 1 hour, less than 6 hours, or less than 48 hours following the start of reperfusion. In some embodiments, treatment is administered immediately after, or about 5, 10, 20, 30, 40, or 50 minutes, or 1, 2, 3, 4, 5, 6, 7, 8, 9, 10, 11, 12, 13, 14, 15, 16, 17, 18, 19, 20, 21, 22, 23, 24, 36, 48, 72, 96, 120, or 240 hours following the start of reperfusion (or at any time in between). In some embodiments, treatment occurs no later than about 240 hours following the start of reperfusion, or no more than about 48 hours following the start of reperfusion, or no more than about 6 hours following the start of reperfusion, or no more than about 1 hour following the start of reperfusion.

In some embodiments, administration of at least one C1-INH and at least one FXII inhibitor can be done prophylactically to prevent initial injury in a patient at risk for a disorder of the contact activation system (e.g., to prevent thrombus formation in a patient at risk for thrombus formation). Prophylactic treatment can be done in a single dose or in repeated doses, such as a dose every 10 minutes, every 30 minutes, every hour, every 6 hours, every 12 hours, every 24 hours, every 36 hours, every 48 hours, every 72 hours, every 96 hours, every 120 hours, every 240 hours, every week, every two weeks, every month, or bimonthly, or any time period in between. Periodic doses can be administered for a set duration of time, for example over the duration of a course of treatment. For example, patients undergoing certain medical procedures may be at increased risk for thrombus formation. Examples of procedures with a risk of thrombotic events include procedures involving extracorporeal blood circulation, invasive diagnostic procedures or surgical procedures including transplantation.

In some embodiments, treatment is administered during certain procedures to reduce the risk of thrombus formation that could result in tissue or organ damage, or is administered to treat thrombotic events occurring during these procedures, e.g., during surgical procedures and other invasive diagnostic procedures, or during extracorporeal circulation. In some embodiments, treatment is administered after certain procedures to reduce the risk of thrombus formation, or to treat thrombotic events occurring after these procedures, e.g., after surgery, after the start of a reperfusion procedure, after thrombolysis, or after extracorporeal blood circulation. Administration after a procedure can be done in a single dose or in multiple doses, e.g., 1, 2, 3, 4, 5 or more doses. Repeated doses can be administered every half hour, every hour, every 6 hours, every 12 hours, every 24 hours, every 36 hours, every 48 hours, every 72 hours, every 96 hours, every 120 hours, or every 240 hours, every week, every two weeks, every month, or bimonthly (or any time period in between) until the completion of the administration protocol.

In some embodiments, administration of at least one C1-INH and at least one FXII inhibitor can be done repeatedly to treat a patient at risk for a disorder of the contact activation system (e.g., to treat a chronic disorder). Such treatment can be done in multiple doses, for example in two, three, four, five, or more doses in a repeated way, such as a dose every 12 hours, every 24 hours, every 48 hours, every 72 hours, every 96 hours, every 120 hours, every 240 hours, every week, every two weeks, every month, bimonthly, or any time period in between. Administration of at least one C1-INH and at least one FXII inhibitor (e.g., to a patient in need of treatment) may occur in a single dose or in repeated administrations, and in any of a variety of physiologically acceptable salt forms, and/or with an acceptable pharmaceutical carrier and/or additive as part of a pharmaceutical composition. Therefore, in certain embodiments, the at least one C1-INH and the at least one FXII inhibitor are administered (i) once, each as a separate injection or infusion, or in a single combined injection or infusion, (ii) in multiple doses of each component or in multiple doses of a combined product, for example in two, three, four, five, or more doses, each as an injection or infusion, or (iii) as an infusion or application. The infusion/application can be administered over a period of time, preferably over a period of 30 minutes to 2 weeks, or 30 minutes to 1 week, or 30 minutes to 6 days, or 30 minutes to 5 days, or 30 minutes to 4 days, or 30 minutes to 3 days, or 30 minutes to 2 days, or 30 minutes to 1 day, or 30 minutes to 12 hours, or 30 minutes to 6 hours (or any time period in between). In an embodiment where the disorder involves a thrombus and/or an IRI injury, the administration may occur in a double dose, once after the initial insult but before the start of reperfusion, and once after the start of reperfusion following the initial injury. In some embodiments, the administration may prevent the formation and/or reduce the size of a secondary edema of the central nervous system, e.g., in a patient who has or has had a brain or spinal cord injury or disease.

The composition comprising at least one C1-INH and the composition comprising at least one FXII inhibitor may be administered to a patient in therapeutically effective amounts. Generally, a therapeutically effective amount may vary with the subject's age, general condition, and gender, as well as the severity of the medical condition in the subject. The dosage may be determined by a physician and adjusted, as necessary, to suit the observed effects of the treatment.

A therapeutically effective dose of the at least one C1-INH may depend on many factors such as, e.g., the type of inhibitor used, the indication, formulation, or mode of administration, and may be determined in preclinical and clinical trials for each respective indication. For example, the dose may range from approximately 0.01 IU/kg to 5000 IU/kg of bodyweight, or from approximately 0.01-1000 IU/kg, or from approximately 5 to 500 IU/kg, or from approximately 10 to 200 IU/kg, or from approximately 20 to 100 IU/kg. In various embodiments, the dose of C1-INH is approximately 0.01, 0.1, 0.5, 1, 5, 10, 15, 20, 25, 30, 50, 75, 100, 125, 150, 175, 200, 225, 250, 275, 300, 325, 350, 375, 400, 450, 500, 1000, 1500, 2000, 2500, 3000, 3500, 4000, 4500, or 5000 IU/kg of bodyweight (or any value in between). A depository may also be used that continuously releases the C1-INH at a suitable rate. Exemplary therapeutic ranges for the C1-INH administration are also disclosed in U.S. Patent 5,939,389.

A therapeutically effective dose of the at least one FXII inhibitor may depend on many factors such as, e.g., the indication, formulation, or mode of administration, and may be determined in preclinical and clinical trials for each respective indication. For example, in one embodiment, the dose of FXII inhibitor is about 0.01, 0.1, 1, 50, 100, 200, 500, or 1000 mg/kg bodyweight, or any dose in between, or ranging from about 0.01-1000 mg/kg, about 0.1-1000, about 1-1000 mg/kg, about 1-500 mg/kg, about 10-200 mg/kg, about 10-100 mg/kg, about 50-500 mg/kg, about 50-200 mg/kg, or about 100-200 mg/kg, or any dose range in between. In certain embodiments, the FXII inhibitor is rHA-Infestin-4. In some embodiments, when rHA-Infestin-4 is used, the dose may range between about 0.01 and 1000 mg/kg body weight, or between about 1 and 1000 mg/kg, or between about 1 and 500 mg/kg, or between about 50 and 500 mg/kg.

In some embodiments, the FXII inhibitor can be an anti-FXII antibody. In those embodiments involving a therapeutically effective dose of an anti-FXII antibody, a therapeutically effective dose is a dose that brings about a positive therapeutic effect in the patient or subject requiring the treatment. A therapeutically effective dose can be in the range of about 0.001 to 100 mg/kg body weight, or from about 0.01 to 100 mg/kg, from about 0.01 to 50 mg/kg, from about 0.1 to 30 mg/kg, from about 0.1 to 10 mg/kg, from about 0.1 to 5 mg/kg, from about 0.1 to 2 mg/kg or from about 0.1 to 1 mg/kg, or any dose range in between. For example, a therapeutically effective dose may be a dose that inhibits FXIIa activity in the subject by at least about 50%, or at least 60%, 70%, 80%, 90%, 95%, 99% or 100% (or any percentage in between).

The administered pharmaceutical compositions may comprise at least one C1-INH and/or at least one FXII inhibitor as the sole active compound(s), or may be delivered in combination with one or more additional compounds, compositions, or biological materials. Examples of additional compounds include vitamins, antibiotics, and compounds intended to remove or inhibit blood clot formation in the brain (e.g., heparin, acetylsalicylic acid, clopidogrel, or dipyridamole).

In certain embodiments, treatment of a disorder of the contact activation system using at least one C1-INH and at least one FXII inhibitor can potentiate the therapeutic effect of either the C1-INH or Factor XII inhibitor when administered as monotherapy. In some embodiments, treatment of a disorder of the contact activation system using at least one C1-INH and at least one FXII inhibitor can produce an additive effect e.g., an effect equivalent to that observed from the sum of the two monotherapies. In some embodiments, the combination acts synergistically to enhance the effect seen from the sum of the two monotherapies. In some embodiments, the use of a C1-INH and a FXII inhibitor enables reduced concentration doses or a less frequent administration schedule of either or both components while achieving a comparable or greater therapeutic effect to that observed with a higher dose concentration or more frequent administration of either component when administered as a monotherapy.

For example, where at least one C1-INH and at least one FXII inhibitor is used, e.g., to reduce the incidence of thrombotic events in one or more vessels, the percentage of a vessel's cross-sectional diameter that is occluded, or the number of vessels that are partially or completely occluded, can be measured before and after treatment. Measurement of occlusion can be done using any standard methods known in the art, e.g., using ultrasound doppler, laser doppler flowmetry, MRI (wash-in, wash-out kinetics for tracers), strain gage plethysmography, impedance plethysmography, photoelectric plethysmography, thermal convection probes, or other suitable techniques for measuring blood flow, or using standard clinical neurological or psychological measures. The reduction (e.g., measured as a percentage of a vessel that is occluded or the percentage of sampled vessels that are partially or completely occluded) using combination therapy can be compared to the percentage reduction in occlusion in a comparable patient or in the same patient using only a C1-INH or a FXII inhibitor. In some embodiments, the reduction in occlusion using combination therapy is additive, (e.g., approximately equivalent reduction to that observed from the sum of the individual treatments).

In some embodiments, the reduction in occlusion observed when using at least one C1-INH and at least one FXII inhibitor therapy is synergistic (e.g., greater than the reduction observed from the sum of the monotherapy effects). In certain embodiments, the combination can increase the reduction observed with C1-INH therapy alone by at least about 5%, 10%, 15%, 20%, 25%, 30%, 35%, 40%, 45%, 50%, 55%, 60%, 65%, 70%, 75%, 80%, 85%, 90%, 95%, 100%, 105%, 110%, 115%, 120%, 125%, 150%, 175%, 200%, 225%, 250%, 275%, or 300% (or any percent in between) or can even lead to a complete reduction, i.e., no occlusion is observed. The concentration of the C1-INH dose in the combination therapy may impact the percent reduction that is observed. In some embodiments, combination therapy can increase the reduction observed with FXII inhibitor therapy alone by at least about 5%, 10%, 15%, 20%, 25%, 30%, 35%, 40%, 45%, 50%, 55%, 60%, 65%, 70%, 75%, 80%, 85%, 90%, 95%, 100%, 105%, 110%, 115%, 120%, 125%, 150%, 175%, 200%, 225%, 250%, 275%, or 300% (or any percent in between) or can even lead to a complete reduction, i.e., no occlusion is observed. The concentration of the FXII inhibitor dose in the combination therapy may impact the percent reduction that is observed.

In certain embodiments the at least one FXII inhibitor and/or the at least one C1 INH are administered at a concentration that produces a reduction in at least one symptom of the disorder of the contact activation system approximately equivalent to an additive effect of each of the FXII inhibitor and the C1 INH when administered separately.

Alternatively in certain embodiments the at least one FXII inhibitor and/or the at least one C1 INH are administered at a concentration that produces a reduction in at least one symptom of the disorder of the contact activation system greater than the additive effect of each of the FXII inhibitor and the C1 INH when administered separately.

In certain embodiments, administration of at least one C1-INH and at least one FXII inhibitor produces a doubling or tripling of the reduction in the percent vessel occlusion observed when a C1-INH is administered on its own following formation of a thrombus. In some embodiments, administration of at least one C1-INH and at least one FXII inhibitor produces a doubling or tripling of the reduction in percent vessel occlusion observed when a FXII inhibitor is administered on its own following formation of a thrombus.

In some embodiments, the effects of treatment with at least one C1-INH and at least one FXII inhibitor on a disorder of the contact activation pathway can be monitored by measuring the extent of tissue damage and/or secondary edema (e.g., after an infarction, stroke, or IRI). Methods for measuring the extent of tissue damage (e.g., brain tissue damage following stroke or heart tissue damage following infarction) can include, e.g., CT scan, MRI, ultrasound, and/or arteriography. In some embodiments, the tissue damage is brain tissue, and the extent of tissue damage can be measured using clinical neurological assessment of brain function. In some embodiments, the extent of tissue damage and/or secondary edema following treatment with at least one C1-INH and at least one FXII inhibitor is reduced by an amount approximately equivalent to that observed from the sum of the individual monotherapies, as compared to tissue damage and/or edema in the absence of any treatment. In some embodiments, the extent of tissue damage and/or secondary edema is reduced by an amount greater than that observed from the sum of the individual monotherapies.

In certain embodiments, the at least one C1-INH and at least one FXII inhibitor are administered at reduced dosages, as compared to the optimal concentration used for monotherapy. In these embodiments, the additive or synergistic effects of combination therapy enable the use of reduced concentrations while maintaining therapeutic effect, thereby reducing cost and/or the risk of adverse reaction to the therapeutic agents (e.g., an immunologic response).

In certain embodiments of the disclosed methods, patients should be treated according to the established standards of care for their clinical presentation. In various embodiments where the patient is a stroke patient, the patient should be examined by MRI, if possible, or other methods to determine whether the brain injury is ischemic or hemorrhagic.

### II. Factor XII Inhibitors

As discussed above the terms "Factor XII" and "FXII" each refer to either or both of Factor XII (e.g., the zymogen or precursor form of the peptide) and activated Factor XII (FXIIa). Thus, "FXII inhibitors" can include inhibitors of either or both of FXII and FXIIa (also termed αFXIIa), including the FXIIa cleavage products FXIIa alpha and FXIIa beta (also termed FXIIf). Further, anti-FXII antibodies include antibodies that bind to and inhibit either or both of FXII and FXIIa. The term "FXII inhibitor" is also meant to include an inhibitor of FXII that is linked to a half-life extending polypeptide, which in some embodiments includes a linker. Examples of FXII inhibitors that can be used include rHA-Infestin-4, anti-FXII antibodies, including modified versions/fragments of these proteins that retain the ability to inhibit the activity of FXII. In an embodiment, the C1-INH is BERINERT®. In an embodiment, the FXII inhibitor is Infestin-4 or a modified Infestin-4 and the C1-INH is BERINERT®. In an embodiment, the FXII inhibitor is an anti-FXII antibody and the C1-INH is BERINERT®.

In some embodiments, the FXII inhibitor is a direct inhibitor of FXII. The term "direct" inhibitor means an inhibitor that acts via contact (e.g., binding) with FXII (or FXIIa), i.e., the FXII inhibitor binds to FXII and/or FXIIa and inhibits its activity and/or activation. In contrast, an indirect inhibitor may act without contacting FXII (or FXIIa) protein. For example, antisense RNA can be used to decrease expression of the FXII gene, or a small molecule can inhibit the effects of FXIIa via interactions with downstream FXIIa reaction partners like Factor XI; these do not interact directly with the FXII protein. Thus, an indirect inhibitor, in contrast to a direct inhibitor, acts upstream or downstream from the FXII protein. Some examples of direct inhibitors are presented below. In some embodiments, the FXII inhibitors are non-endogenous inhibitors; that is, they are not inhibitors that occur naturally in the human or animal body.

C1-INH is also a weak FXII inhibitor. However, the FXII inhibitor used in the combination therapy is an inhibitor other than C1-INH.

In one embodiment the FXII inhibitor is a specific FXII inhibitor, preferably a specific FXIIa inhibitor.

A specific FXII inhibitor refers to an inhibitor which inhibits plasmatic serine proteases or other endogenous proteins other than FXII and/or FXIIa less than or equal to 25% if used in a molar ratio of 1:1. In other words: a specific FXII/FXIIa inhibitor inhibits plasmatic serine proteases other than FXII and/or FXIIa less than or equal to 25% when said inhibitor is used in a molar ratio of 1:1 of the respective plasmatic serine protease to said inhibitor. Preferably the FXII inhibitor inhibits plasmatic serine proteases other than FXII and/or FXIIa less than or equal to 20%, preferably less than or equal to 15%, preferably less than or equal to 10%, preferably less than or equal to 5%, preferably less than or equal to 1% if used in a molar ratio of 1:1. For example, a specific FXII mAb inhibits the plasmatic serine protease FXIa by only 5%, wherein the molar ratio of FXIa to said mAb is 1:1 whereas the same FXII mAb inhibits FXIIa by at least 80%, preferably at least 90%.

In one embodiment of the invention one other plasmatic serine protease is inhibited by more than 50% if used in a molar ratio of 1:1 of the respective plasmatic serine protease to said inhibitor.

In another embodiment of the invention two other plasmatic serine proteases are inhibited by more than 50% if used in a molar ratio of 1:1 of the respective plasmatic serine protease to said inhibitor.

In yet another embodiment the FXII inhibitor is a human FXII inhibitor, including a humanized monoclonal antibody, preferably a fully human monoclonal antibody.

"Homology" as used herein refers to the percentage number of amino acids that are identical or constitute conservative substitutions. Homology may be determined using sequence comparison programs such as GAP (Deveraux et al., 1984, Nucleic Acids Research 12, 387-395). In this way sequences of a similar or substantially different length to those cited herein could be compared by insertion of gaps into the alignment, such gaps being determined, for example, by the comparison algorithm used by GAP.

### A. Infestin-4

In one embodiment, the disclosure provides a FXII inhibitor comprising infestin domain 4 (referred to as "Infestin-4"). Infestins are a class of serine protease inhibitors derived from the midgut of the hematophagous insect, *Triatoma infestans,* a major vector for the parasite *Trypanosoma cruzi,* known to cause Chagas disease. (Campos ITN et al. 32 Insect Biochem. Mol. Bio. 991-997, 2002; Campos ITN et al. 577 FEBS Lett. 512-516, 2004; WO 2008/098720.) This insect uses these inhibitors to prevent coagulation of ingested blood. The infestin gene encodes 4 domains that result in proteins that can inhibit different factors in the coagulation pathway. In particular, domain 4 encodes a protein (Infestin-4) that is a strong inhibitor of FXIIa. Infestin-4 has been administered in mice without resulting in bleeding complications. (WO 2008/098720; Hagedorn et al., Circulation 2010; 121:1510-17.)

In various embodiments, a FXII inhibitor comprises Infestin-4. The term "Infestin-4," as used herein, encompasses variants or fragments of the wild-type peptide that retain the ability to inhibit FXII. In some embodiments, the Infestin-4 is chosen for its ability to inhibit FXIIa. In certain embodiments, the Infestin-4 comprises a variant of Infestin-4, wherein the variant comprises Infestin domain 4, and optionally, Infestin domains 1, 2, and/or 3. In one embodiment, the Infestin-4 is a (His)₆-tagged Infestin-4 construct. In another embodiment, the Infestin-4 is a fusion protein comprising a fusion partner, such as a half-life enhancing polypeptide (e.g., albumin, an Fc domain of an IgG, or PEG), bound to infestin-4. In some embodiments, a linker connects the fusion partner to Infestin-4. In various embodiments, the Infestin-4 is the rHA-Infestin-4 protein described in Hagedorn et al., Circulation 2010; 117:1153-60. In one embodiment, a composition comprises albumin bound to the rHA-Infestin-4 protein described in Hagedorn et al. by a flexible linker. In certain embodiments, other Infestin-4 inhibitors of FXII are used, examples of which are described in WO 2008/098720 and Hagedorn et al., Circulation 2010; 117:1153-60.

An example of a wild type Infestin-4 sequence is presented in SEQ ID NO: 1: EVRNPCACFRNYVPVCGSDGKTYGNPCMLNCAAQTKVPGLKLVHEGRC.

As used here, the term "variant" of Infestin-4 refers to a polypeptide with one or more amino acid mutation, wherein "mutation" is defined as a substitution, a deletion, or an addition, to the wild type Infestin-4 sequence (SEQ ID NO: 1). The term "Infestin-4" encompasses these Infestin-4 variants. The term "variant" of Infestin-4 also includes fragments of the wild type or a mutated Infestin-4 sequence. In various embodiments, the one or more mutations to the wild type Infestin-4 sequence do not substantially alter the functional ability of the polypeptide to inhibit FXII. In some embodiments, the one or more mutations do not completely or substantially remove the ability of the polypeptide to inhibit FXII (e.g., the variant retains at least about 20%, 30%, 40%, 50%, 60%, 70%, 80%, 90%, 95%, 98%, 99%, or more of the inhibitory ability of wild type Infestin-4). Further examples of such variants are provided below.

In one embodiment, an Infestin-4 variant comprises the amino acid sequence VRNPCACFRNYV (SEQ ID NO: 20, which are residues 2-13 of SEQ ID NO: 1) from the amino terminal of the wild type Infestin-4 sequence. In certain embodiments, the variant can comprise residues 2-13 of SEQ ID NO: 1 and also comprises at least one, and optionally up to five, amino acid mutations, as compared to the wild type Infestin-4 sequence, outside residues 2-13 of SEQ ID NO: 1. In some embodiments, the variant retains six conserved cysteine residues from the wild type Infestin-4 sequence, and/or a homology of at least 70%, at least 75%, at least 80%, at least 85%, at least 90%, at least 95%, at least 98%, or at least 99%, to the wild type Infestin-4 sequence. In some embodiments, a variant of Infestin-4 comprises the conserved N-terminal region amino acids 2-13 of the wild type Infestin-4 sequence, and at least one, and optionally up to five, amino acid mutations outside these conserved N-terminal amino acids, resulting in differences from the wild type Infestin-4 sequence. As used here, the term "outside the N-terminal amino acids" of an Infestin variant refers to any amino acid along the polypeptide chain of the variant other than the contiguous stretch of amino acids that comprises the sequence of SEQ ID NO: 20: VRNPCACFRNYV, which are amino acids 2-13 from SEQ ID NO: 1.

In another embodiment, an Infestin-4 variant comprises six conserved cysteine residues and/or has a homology of at least 70%, at least 75%, at least 80%, at least 85%, at least 90%, at least 95%, at least 98%, or at least 99%, to the wild type Infestin-4 sequence. In one embodiment, the six conserved cysteine residues are amino acids at positions 6, 8, 16, 27, 31, and 48 of the wild type Infestin-4 sequence, SEQ ID NO: 1. In one embodiment, the variant comprises the final conserved cysteine at position 48. In other embodiments, the exact positions of the cysteine residues, and relative positions to each other, may change from positions 6, 8, 16, 27, 31, and 48 of the wild type Infestin-4 sequence due to insertions or deletions in the Infestin-4 variant. Nevertheless, in these embodiments, an Infestin-4 variant comprises all six cysteines and/or may share 70%, 75%, 85%, 90%, 91%, 92%, 93%, 94%, 95%, 96%, 97%, 98% or 99%, or any percentage in between homology to the wild type Infestin-4 sequence. In some embodiments, the Infestin-4 variant retains amino acids 2-13 from SEQ ID NO: 1 as well as all six cysteine residues, and may share 70%, 75%, 85%, 90%, 91%, 92%, 93%, 94%, 95%, 96%, 97%, 98% or 99%, or any percentage in between homology to the wild type Infestin-4 sequence.

In some embodiments, the Infestin-4 variant comprises SEQ ID NO: 21: DSLGREVRNPCA. In some embodiments, this sequence is added at or near the N-terminus of a fragment or full length wild type Infestin-4 sequence and derives from the human protein SPINK-1.
In some embodiments, an Infestin-4 variant comprises a fusion construct between wild-type Infestin-4 or a variant Infestin-4 and human albumin (referred to as "HA"). In some embodiments, the HA is a recombinant protein (referred to as "rHA"). In certain embodiments, the Infestin-4 and HA proteins are joined directly, or via a linker polypeptide.

In one embodiment, the FXII inhibitor comprises a variant of the wild type Infestin-4 polypeptide sequence, wherein the variant comprises the N-terminal amino acids 2-13 of SEQ ID NO: 1; at least one, and optionally up to five, amino acid mutations outside the N-terminal amino acids; six conserved cysteine residues; and/or homology of at least 70%, at least 75%, at least 80%, at least 85%, at least 90%, at least 95%, at least 98%, or at least 99%, to the wild type Infestin-4 sequence.

In various embodiments, a variant of Infestin-4 is provided that retains the ability to inhibit FXII. The functional inhibitory activity may be assessed, for example, through *in vitro* and/or *in vivo* characterization, including direct assays to test inhibition of FXII enzyme activity, prolonged coagulation time (e.g., activated partial thromboplastin time, aPTT), clinical clotting tests that address the intrinsic pathway of coagulation, or *in vivo* methods that evaluate coagulation.

Further examples of Infestin-4 variants are SPINK-1 mutants, which are described below.

### B. FXII Antibodies

In various embodiments, the FXII inhibitor is an anti-FXII antibody that binds to FXII and inhibits or reduces FXII activation and/or activity. The term "anti-factor XII antibody" encompasses full length antibodies and functional fragments thereof (e.g., antigen binding fragments such as Fab, F(ab)₂, Fv, and scFv). The term also encompasses polyclonal and monoclonal antibodies and antibodies of any of the isotypes such as IgM, IgD, IgA, IgG, and IgE, and any subclass thereof, such as IgG₁, The antibody may be from a mammalian species such as human, mouse, rat, rabbit, goat, hamster, or monkey. In some embodiments, the antibody may be humanized or CDR-grafted. In some embodiments, the antibody may be mutated or modified to alter immunogenicity, half-life, and/or to impart other advantageous properties associated with a therapeutic antibody. In one embodiment, the antibody is an anti-FXII antibody that binds to an epitope on the heavy chain or light chain of FXII, such as a neutralizing epitope. In some embodiments, the antibody may be conjugated to a polypeptide, nucleic acid, or small molecule. An "anti-FXII antibody" also includes antibodies that bind to and/or inhibit either or both of the zymogen of FXII and the activated protein (FXIIa), including the FXIIa alpha and FXIIa beta cleavage fragments. In some embodiments, the antibody binds specifically to FXIIa or the alpha or beta chain fragments of FXIIa.

The anti-FXII antibody binds to and inhibits FXIIa activation and/or activity. Anti-FXII antibodies have been described, for example, in WO 2006/066878, and in Ravon et al., Blood 86: 4134-43 (1995). Other monoclonal antibodies (mAbs) to human Factor XII include the B7C9 mAb described by Pixley et al. (J Biol Chem 1987; 262, 10140-45); a mAb described by Small et al. (Blood 1985; 65:202-10); the monoclonal antibodies F1 and F3 described by Nuijens et al. (J. Biol. Chem. 1989; 264:12941-49); the B6F5, C6B7, and D2E10 monoclonal antibodies against the light chain of FXII described in WO89/11865; a monoclonal antibody that selectively binds FXIIa-β over FXII described in WO90/08835; and the anti-FXII antibody OT-2 described in WO91/17258.

Additional anti-Factor XII monoclonal antibodies are described in WO 2013/014092 A1. In some embodiments, the antibodies may have a more than 2 fold higher binding affinity to human Factor XIIa-beta than to unactivated human FXII and may be capable of inhibiting the amidolytic activity of human Factor Xlla.

In certain embodiments, an anti-FXII antibody comprises the heavy chain variable region (VH) and light chain variable region (VL) sequences presented in Table 1. In some embodiments, an anti-FXII antibody comprises the HCDR1, HCDR2, and HCDR3, and/or comprises the VCDR1, VCDR2, and VCDR3 shown in Table 1. Antibody 3F7 as described in WO 2013/014092 A1 is an example of such an antibody.

**Table 1.**

| **Region** | **Amino acid sequence** |
|---|---|
| VH | |
| VL | |
| HCDR 1 *(Kabat 31-35)* | KYIMQ (SEQ ID NO: 8) |
| HCDR 2 *(Kabat 50-65)* | GIRPSGGTTVYADSVKG (SEQ ID NO: 9) |
| HCDR 3 *(Kabat 95-102)* | ALPRSGYLISPHYYYYALDV (SEQ ID NO: 11) |
| LCDR 1 *(Kabat 24-34)* | SGSSSNIGRNYVY (SEQ ID NO: 13) |
| LCDR 2 *(Kabat 50-56)* | SNNQRPS (SEQ ID NO: 14) |
| LCDR 3 *(Kabat 89-97)* | AAWDASLRGV (SEQ ID NO: 15) |

In some embodiments, the antibody has one or more of the following features: (a) binds human FXII; (b) comprises a heavy chain variable (VH) region which is more than 85% identical to the sequence of SEQ ID NO: 6, such as more than 90%, 95%, 98%, or 99% identical; (c) comprises a light chain variable (VL) region which is more than 85% identical to the sequence of SEQ ID NO: 7, such as more than 90%, 95%, 98%, or 99% identical; (d) comprises heavy chain CDR1 at least 80% identical to the sequence of SEQ ID NO: 8, such as more than 85%, 90%, 95%, 98%, or 99% identical, and/or heavy chain CDR2 at least 60% identical with SEQ ID NO: 9, such as more than 70%, 80%, 85%, 90%, 95%, 98%, or 99% identical, and/or heavy chain CDR3 at least 80% identical to the sequence of SEQ ID NO: 11, such as more than 85%, 90%, 95%, 98%, or 99% identical; (e) comprises light chain CDR1 at least 50% identical to SEQ ID NO: 13, such as more than 60%, 70%, 80%, 85%, 90%, 95%, 98%, or 99% identical, and/or at least 50% identical to light chain CDR2 of SEQ ID NO: 14, such as more than 60%, 70%, 80%, 85%, 90%, 95%, 98%, or 99% identical,, and/or at least 50% identical to light chain CDR3, with the sequence A-X₁-W-X₂-X₃-X₄-X₅-R-X₆-X₇ (SEQ ID NO: 16), such as more than 60%, 70%, 80%, 85%, 90%, 95%, 98%, or 99% identical; wherein X₁ can be A or S, X₅ can be L or V, and the other X_{n'}'s can be any amino acid; (f) binds human Factor XIIa-beta with a K_{D} of better than 10⁻⁸M; (g) competes with Infestin-4 for binding to human Factor XIIa-beta; or (h) is a human IgG or functional variant thereof, preferably human IgG4 or a functional variant thereof.

In certain embodiments, the anti-FXII antibody is an IgG antibody that binds human FXII/FXIIa and comprises (a) a VH region comprising heavy chain CDR1 as set forth in SEQ ID NO: 8, heavy chain CDR2 as set forth in SEQ ID NO: 10, and heavy chain CDR3 as set forth in SEQ ID NO: 12; and/or (b) a VL region comprising light chain CDR1 as set forth in SEQ ID NO: 13, light chain CDR2 as set forth in SEQ ID NO: 14, and light chain CDR3 as set forth in SEQ ID NO: 16. A heavy chain CDR2 can comprise the sequence GIX₁X₂X₃X₄X₅X₆TVYADSVKG (SEQ ID NO: 10), wherein X₁ is R, N or D, X₂ is P, V, I, or M; X₃ is S, P, or A; X₄ is G, L, V, or T; X₅ can be any amino acid, preferably X₅ is G, Y, Q, K, R, N, or M; and X₆ is T, G, or S. A heavy chain CDR3 can comprise the sequence ALPRSGYLX₁X₂X₃X₄YYYYALDV (SEQ ID NO: 12), wherein X₁ is I, M or V; X₂ is S or K; X₃ is P, K, T, or H; and X₄ is H, N, G, or Q. A light chain CDR3 can comprise the sequence AX₁WX₂X₃X₄X₅RX₆X₇ (SEQ ID NO: 16), wherein X₁ is A or S; X₂ is D, Y, E, T, W, E, or S; X₃ is A, N, I, L, V, P, Q, or E; X₄ is S, D, P, E, Q, or R; X₅ is L or V; X₆ is G, L, or K; and X₇ is V, A, D, T, M, or G.

In other embodiments, the anti-FXII antibody is a fragment of an IgG antibody that binds human FXII/FXIIa and comprises (a) a VH region comprising heavy chain CDR1 as set forth in SEQ ID NO: 8, heavy chain CDR2 as set forth in SEQ ID NO: 9, and heavy chain CDR3 as set forth in SEQ ID NO: 11; and/or (b) a VL region comprising light chain CDR1 as set forth in SEQ ID NO: 13, light chain CDR2 as set forth in SEQ ID NO: 14, and light chain CDR3 as set forth in SEQ ID NO: 15.

In various embodiments, the anti-FXII antibody is an affinity matured, chimeric, CDR grafted, or humanized antibody, or a functional antigen binding fragment thereof. In some embodiments, the anti-FXII antibody is chosen from the affinity matured (relative to 3F7) antibodies VR115, VR112, VR24, VR110, and VR119 (SEQ ID NOs for HCDR 1-3 and LCDR1-3 of these antibodies are shown below in Table 2).

**Table 2.**

| **mAb** | **HCDR1** | **HCDR2** | **HCDR3** | **LCDR1** | **LCDR2** | **LCDR3** |
|---|---|---|---|---|---|---|
| 3F7 | 8 | 9 | 11 | 13 | 14 | 15 |
| VR119 | 8 | 10 | 11 | 13 | 14 | 15 |
| VR112 | 8 | 10 | 11 | 13 | 14 | 15 |
| VR115 | 8 | 10 | 11 | 13 | 14 | 15 |
| VR24 | 8 | 9 | 11 | 17 | 14 | 15 |
| VR110 | 8 | 10 | 11 | 13 | 14 | 15 |

As noted above, SEQ ID NO: 10 is a degenerate sequence. VR119 comprises SEQ ID NO: 10 wherein X₁ is N, X₂ is V, X₃ is P; X₄ is L, X₅ Y; and X₆ is G. VR112 comprises SEQ ID NO: 10 wherein X₁ is N, X₂ is V, X₃ is P, X₄ is V, X₅ is Q, and X₆ is G. VR115 comprises SEQ ID NO: 10 wherein X₁ is D, X₂ is I, X₃ is P, X₄ is T, X₅ is K, and X₆ is G. VR110 comprises SEQ ID NO: 10 wherein X₁ is D, X₂ is M, X₃ is P, X₄ is T, X₅ is K, and X₆ is G. VR24 comprises a unique LCDR1: SGSSEMTVHHYVY (SEQ ID NO: 17).

In embodiments involving antibody CDRs, CDR's are defined according to the KABAT numbering system. (Kabat et al., Sequences of proteins of immunological interest, 5th edn. U.S. Department of Health and Human services, NIH, Bethesda, MD. (1991))

In some embodiments, the antibody is an anti-FXII monoclonal antibody or antigen-binding fragment thereof that inhibits human FXIIa-alpha, e.g., in an in vitro FXIIa amidolytic activity assay (WO 2013/014092), by more than 40%, more than 50%, or more than 60%, when used at a molar ratio of 1:0.2 of FXIIa-alpha to antibody. In some embodiments, the antibody or antigen binding fragment thereof inhibits human Factor XIIa-alpha by more than 80%, more than 85%, or more than 90%, when used at a molar ratio of 1:0.5 of FXIIa-alpha to antibody. In one embodiment, the antibody achieves complete or nearly complete (e.g., 95%, 96%, 97%, 98%, 99%, or greater) inhibition of human FXIIa-alpha when used at a molar ratio of 1:0.5. In one embodiment, the antibody or antigen binding fragment thereof has an affinity for human FXIIa that is at least approximately comparable to that of antibody 3F7.

### C. FXII Inhibitors linked to HLEPs

Another aspect of the disclosure provides FXII inhibitors linked to fusion partners, such as a half-life enhancing polypeptides (HLEPs) or molecules such as PEG. In one embodiment, FXII inhibitors are small proteins. Therefore, rapid renal clearance (as is observed for other small proteins) can be expected (See Werle M and Bernkop-Schnurch A, Amino Acids 2006; 30:351-367). One way to address a short plasma half-life of a polypeptidic compound is to inject it repeatedly or via continuous infusion. Another approach is to increase the intrinsic plasma half-life of the polypeptide itself. For example, in one embodiment, FXII inhibitors are linked to half-life extending proteins.

A "half-life enhancing polypeptide" is a polypeptide fusion partner that may increase the half-life of the FXII inhibitor *in vivo* in a patient or in an animal. Examples include albumin and immunoglobulins and their fragments, such as Fc domains, or derivatives, which may be fused to a FXII inhibitor directly or via a cleavable or non-cleavable linker. Ballance et al. (WO 2001/79271) described fusion polypeptides comprising a multitude of different therapeutic polypeptides fused to human serum albumin.

The terms "albumin" and "serum albumin" encompass human albumin (HA) and variants thereof, the full mature form of which is disclosed herein (SEQ ID NO: 19), as well as albumin from other species and variants thereof. As used herein, "albumin" refers to an albumin polypeptide or amino acid sequence, or an albumin variant, having one or more functional activities (e.g. biological activities) of albumin. In certain embodiments, albumin is used to stabilize or prolong the therapeutic activity of a FXII inhibitor. The albumin may be derived from any vertebrate, especially any mammal, for example human, monkey, cow, sheep, or pig. Non-mammalian albumin can also be used and includes, but is not limited to, albumin from chicken and salmon. The albumin portion of the albumin-linked polypeptide may be from a different animal than the therapeutic polypeptide portion. See WO 2008/098720 for examples of albumin fusion proteins.

In one embodiment, an albumin variant is at least 10, 20, 40, 50, 60, or at least 70 amino acids long (or any length in between) or may include 15, 20, 25, 30, 50 or more contiguous amino acids (or any number in between) from a human albumin (HA) sequence (e.g., SEQ ID NO: 19), or may include part or all of specific domains of HA. An albumin variant may include an amino acid substitution, deletion, or addition, either conservative or non-conservative substitution, wherein such changes do not substantially alter the active site, or active domain, which confers the therapeutic activities of the half-life enhancing polypeptides. These variants may share homology of 70%, 75%, 80%, 85%, 90%, 91%, 92%, 93%, 94%, 95%, 96%, 97%, 98% or 99% (or any percentage in between).

In some embodiments, the albumin variant is a fragment and may comprise at least one whole domain of albumin and/or fragments of those domains, for example domains 1 (amino acids 1-194 of SEQ ID NO: 19), 2 (amino acids 195-387 of SEQ ID NO: 19), 3 (amino acids 388-585 of SEQ ID NO 19), 1 + 2 (1-387 of SEQ ID NO: 19), 2 + 3 (195-585 of SEQ ID NO: 19) or 1 + 3 (amino acids 1-194 + amino acids 388-585 of SEQ ID NO: 19). Each domain is itself made up of two homologous subdomains namely residues 1-105, 120-194, 195-291, 316-387, 388-491 and 512-585, of SEQ ID NO: 19, with flexible inter-subdomain linker regions comprising residues Lys106 to Glu119, Glu292 to Val315 and Glu492 to Ala511. Thus, in some embodiments, the albumin variant comprises at least one whole subdomain of albumin.

In certain embodiments, other proteins that are structurally or evolutionarily related to albumin ("albumin family proteins") may be used as HLEPs, including, but not limited to alpha-fetoprotein (WO 2005/024044; Beattie and Dugaiczyk, 20 Gene 415-422, 1982), afamin (Lichenstein et al. 269 J. Biol. Chem. 18149-18154, 1994), and vitamin D binding protein (Cooke and David, 76 J. Clin. Invest. 2420-2424, 1985). The genes encoding these proteins represent a multigene cluster with structural and functional similarities mapping to the same chromosomal region in humans, mice, and rats. The structural similarity of the albumin family members suggests that they can be used as HLEPs. For example, alpha-fetoprotein has been claimed to extend the half-life of an attached therapeutic polypeptide *in vivo* (WO 2005/024044). Thus, in some embodiments, these proteins, or variants thereof, that may be capable of stabilizing or prolonging therapeutic activity, can be used as HLEPs linked to FXII or FXIIa and may be derived from any vertebrate, especially any mammal, for example human, monkey, cow, sheep, or pig, or non-mammal including but not limited to, hen or salmon. In some embodiments, variants may comprise 10 or more amino acids in length, or may comprise about 15, 20, 25, 30, 50 or more contiguous amino acids of the respective protein sequence from which they are derived, or may include part or all of specific domains of the respective proteins. Albumin family member fusion proteins may include naturally occurring polymorphic variants.

In certain embodiments, mono- or poly- (e.g., 2-4) polyethylene glycol (PEG) moieties may be used as fusion partners and may extend *in vivo* half-lives. Pegylation may be carried out by any of the pegylation reactions available. Exemplary methods for preparing pegylated protein products can generally include (a) reacting a polypeptide with polyethylene glycol (such as a reactive ester or aldehyde derivative of PEG) under conditions whereby the protein becomes attached to one or more PEG groups; and (b) obtaining the reaction product(s). There are a number of PEG attachment methods. See, for example, EP 0 401 384; Malik et al., Exp. Hematol., 20:1028-1035 (1992); Francis, Focus on Growth Factors, 3(2):4-10 (1992); EP 0 154 316; EP 0 401 384; WO 92/16221; WO 95/34326; U.S. Pat. No. 5,252,714.

In some embodiments, an immunoglobulin (Ig) may be used as an HLEP. The term "immunoglobulin" encompasses functional fragments and variants thereof, such as an Fc region or one or more Ig constant domains. In some embodiments, the Ig comprises an Fc region or portions of the immunoglobulin constant domain(s). The constant region may be that of an IgM, IgG, IgD, IgA, or IgE immunoglobulin. In some embodiments, the therapeutic polypeptide portion is connected to the Ig via the hinge region of the antibody or a peptide linker, which may be cleavable. Several patents and patent applications describe the fusion of therapeutic proteins to immunoglobulin constant regions to extend the therapeutic protein's half-life *in vivo* (US 2004/0087778, WO 2005/001025, WO 2005/063808, WO 2003/076567, WO 2005/000892, WO 2004/101740, US 6,403,077). Therefore, in some embodiments, immunoglobulin regions (e.g., Fc domains, Fc fragments of immunoglobulins, and variants thereof) are used as HLEPs. In some embodiments, inhibitors of FXII can be fused to Fc domains or portions of immunoglobulin constant regions as HLEPs. In some embodiments, these fusion proteins are prepared as recombinant molecules expressed in prokaryotic or eukaryotic host cells, such as bacteria, yeast, plant, animal (including insect) or human cell lines or in transgenic animals (WO 2008/098720).

### D. Linkers

In various embodiments, an intervening peptidic linker may be introduced between a therapeutic polypeptide and a HLEP. In one embodiment, a cleavable linker is introduced, particularly if the HLEP has the potential to interfere with the therapeutic polypeptide's specific activity, e.g. by steric hindrance. In certain embodiments, the linker is cleavable by enzymes involved in coagulation, such as coagulation proteases of the intrinsic, extrinsic, or common coagulation pathway. Coagulation proteases of the intrinsic pathway include proteases in the contact activation pathway, e.g., FXIIa, FXIa, or FIXa. In one embodiment, the linker is cleaved by FXIIa. Proteases of the extrinsic pathway include proteases in the tissue factor pathway, for example, FVIIa. Proteases of the common pathway include proteases involved in the conversion of fibrinogen to fibrin, for example, FXa, FIIa, and FXIIIa.

### III. C1 Inhibitor

The terms "C1 Inhibitor," "C1 esterase Inhibitor," and "C1-INH" refer to serine protease inhibitors and functional fragments thereof that bind to and inhibit proteins in the C1 complex, preventing activation of circulating proteases and/or inhibiting activated proteases associated with the complement system, the kallikrein-kinin system, and/or the coagulation system. Coagulation proteases inhibited by C1-INH include Factor XIa and Factor XIIa. C1-INHs are plasmatic (i.e. plasma-derived), recombinant, or a combination of the two. Examples of C1-INH include plasma-derived CINRYZE™ (Viropharma), recombinant RUCONEST™ or RHUCIN™ (both Pharming), and plasma-derived BERINERT® (CSL Behring).

C1-INH as used herein can comprise native serine protease inhibitors from a human or animal, or can comprise recombinant peptides, synthetic peptides, or peptide mimetics. For example, a human plasma-derived C1-INH can be used, e.g., following pasteurization and nanofiltration. An example of an amino acid sequence of human C1-INH is provided in WO 2007/073186 (see SEQ ID NO: 1 in WO 2007/073186). For further disclosure regarding the structure and function of C1-Inhibitor, see U.S. Patent 4,915,945; U.S. Patent 5,939,389; U.S. Patent 6,248,365; U.S. Patent 7,053,176; and WO 2007/073186.

In various embodiments, C1-Inhibitor used in the methods and compositions disclosed herein can be produced according to methods known to one of skill in the art, e.g., to produce recombinant C1-INH. For example, a process for producing C1-inhibitor for therapeutic purposes is disclosed in U.S. Patent 4,915,945. Alternatively, in some embodiments C1-INH can be collected and concentrated from natural tissue sources using techniques known in the art. In some embodiments, recombinant technology can be used to produce C1-INH, such as recombinant human C1-INH, in a transgenic animal or cell line. Commercially available products comprising C1-inhibitor are, e.g. plasma-derived CINRYZE™ (Viropharma), recombinant RUCONEST™ or RHUCIN™ (both Pharming), and plasma-derived BERINERT® (CSL Behring). BERINERT® is indicated for treatment of hereditary angioedema and congenital deficiencies. Recombinant C1-INH can be prepared by known methods.

In some embodiments, one or more human plasma-derived C1-INH is used in the compositions and methods disclosed herein. In some embodiments, one or more recombinant human C1-INH is used. In some embodiments, a combination of one or more human plasma-derived C1-INH and one or more recombinant human C1-INH are used.

### IV. Pharmaceutical Compositions

In any of the various aspects of the invention, the FXII inhibitor and/or C1-INH may have a purity of greater than 80%, or greater than 95%, 96%, 97%, 98%, or 99%. In one embodiment, the FXII inhibitor and/or C1-INH may have a pharmaceutically pure state that is greater than 99.9% pure with respect to contaminating macromolecules, such as other proteins and nucleic acids, and may be free of infectious and pyrogenic agents.

In certain embodiments, a pharmaceutical composition can comprise at least one additive such as a filler, bulking agent, buffer, stabilizer, or excipient. Some exemplary pharmaceutical formulation techniques are described, e.g., in the 2005 Physicians' Desk Reference, Thomson Healthcare: Montvale, NJ, 2004; Remington: The Science and Practice of Pharmacy, 20th ed., Gennado et al., Eds. Lippincott Williams & Wilkins: Philadelphia, PA, 2000; Kibbe et al. Handbook of Pharmaceutical Excipients, (3rd ed., Pharmaceutical Press), 2000.). Pharmaceutical additives include, e.g., mannitol, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. In certain embodiments, the pharmaceutical compositions may also contain pH buffering reagents and wetting or emulsifying agents. In further embodiments, the compositions may contain preservatives and/or stabilizers. Pharmaceutical compositions may be formulated in lyophilized or stable soluble form. Polypeptides may be lyophilized by a variety of procedures known in the art.

In certain embodiments, a pharmaceutical composition comprising at least one C1-INH and/or a pharmaceutical composition comprising at least one FXII inhibitor are prepared for use in treating a disorder of the contact activation system. For example, if a powder or lyophilized form of C1-INH or FXII inhibitor (e.g., by freeze drying) is provided and an aqueous pharmaceutical is desired, the powder can be dissolved by mixing with aqueous components of the pharmaceutical formulation and stirred using suitable techniques such as vortexing or gentle agitation. Alternatively, if an aqueous pharmaceutical is desired and the C1-INH or FXII inhibitor is already in aqueous form, the components can be directly combined prior to administration. In certain embodiments, C1-INH is provided in lyophilized form and combined with an aqueous FXII inhibitor composition prior to administration to a patient. In other embodiments, FXII inhibitor is provided in lyophilized form and combined with aqueous C1-INH composition prior to administration. In still other embodiments, both C1-INH and FXII inhibitor are provided in lyophilized form and combined with aqueous pharmaceutical components (e.g., additional active components or inactive components such as fillers, stabilizers, solvents, or carriers) prior to administration. In yet other embodiments, both C1-INH and FXII inhibitor are provided in aqueous form and may be administered separately or may be combined prior to administration to a patient.

The formulation of pharmaceutical compositions may vary depending on the intended route of administrations and other parameters (see, e.g., Rowe et al., Handbook of Pharmaceutical Excipients, 4th ed., APhA Publications, 2003). In some embodiments, the pharmaceutical composition may be a lyophilized cake or powder. The lyophilized composition may be reconstituted for administration by intravenous injection, for example with Sterile Water for Injection, USP. In other embodiments, the composition may be a sterile, non-pyrogenic solution. In still further embodiments, the composition is delivered in powder form in a pill or tablet.

Formulations of the FXII inhibitor and/or the C1-INH may be delivered to the patient by any pharmaceutically suitable means of administration. For example, the compositions can be administered systemically, such as parenterally. Parenteral formulations may be administered intravenously or subcutaneously, either in bolus form or as an infusion, according to known procedures. Preferred liquid carriers, which are well known for parenteral use, include sterile water, saline, aqueous dextrose, sugar solutions, ethanol, glycols, and oils. For systemic use, the therapeutic proteins may be formulated for an intravenous line or an arterial line. The formulations may be administered continuously by infusion or by bolus injection.

Tablets and capsules for oral or rectal administration may contain conventional excipients such as binding agents, fillers, lubricants, or wetting agents, etc. Oral or rectal liquid preparations may be in the form of aqueous or oily suspensions, solutions, emulsions, syrups, elixirs or the like, or may be presented as a dry product for reconstitution with water or other suitable vehicle prior to use. Such liquid preparations may contain conventional additives, such as suspending agents, emulsifying agents, non-aqueous vehicles, and preservatives. Some formulations encompass slow release systems, such as a patch.

### EXAMPLES

The following examples serve to illustrate, and in no way limit, the present disclosure.

### Example 1: Synergistic antithrombotic effects in FeCl₃-induced arterial thrombosis in rats by a combined intravenous treatment of rHA-Infestin-4 and BERINERT®

The effect of combining treatment with Factor XII inhibitor rHA-Infestin-4 and C1-inhibitor (INH) BERINERT® was evaluated in a rat arterial thrombosis model. Treatment groups were as shown in Table 3:

**Table 3: Treatment groups**

| **No.** | **Treatment** | **Dose** | **Volume** | **Schedul e** | **Route** | **N** |
|---|---|---|---|---|---|---|
| 1 | Isotonic saline | N/A¹ | 4 mL/kg | t=-5 min | i.v. | 11 |
| 2 | rHA-Infestin-4 | 4 mg/kg | 4 mL/kg | t=-5 min | i.v. | 11 |
| 3 | C1-INH (BERINERT®) | 20 IU/kg | 4 mL/kg | t=-5 min | i.v. | 11 |
| 4 | rHA-Infestin-4 + C1-INH (BERINERT®) | 4 mg/kg | 4 mL/kg | t=-5 min | i.v. | 10 |
| | | 20 IU/kg | | | | |

| | | | | | | |
|---|---|---|---|---|---|---|
| ¹N/A = not applicable | | | | | | |

Female CD-Rats (7-8 weeks), obtained from Charles River Laboratories, received a single i.v. injection of the treatment solution as listed in Table 3 at t= -5 min in deep anesthesia. Thereafter, the effects of the treatment on thrombotic occlusion were quantified. Baseline blood flow was determined by placing an ultrasonic flow probe around the exposed carotid artery. To initiate thrombosis, a 2.5 mm² (1×2.5 mm) patch of filter paper saturated with 35 % ferric chloride solution was placed on the carotid artery downstream from the flow probe at t=0 min. After 3 minutes the filter paper was removed and blood flow was monitored for 60 minutes or until thrombotic occlusion of the vessel (i.e., cessation of blood flow).

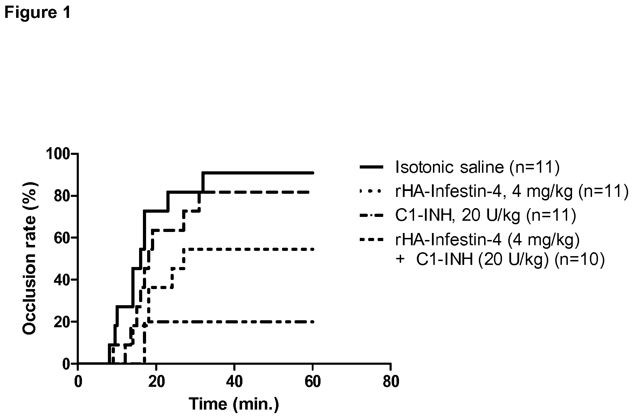

Figure 1 presents the time course of arterial occlusion, as well as occlusion rates following FeCl₃ application. Table 4 presents the occlusion rates (i.e., number of animals where total occlusion was observed) in each treatment group.

**Table 4: Occlusions rates following treatment**

| **No.** | **Treatment** | **Occlusion Rate** | **N** |
|---|---|---|---|
| 1 | Isotonic saline | 10/11 (91 %) | 11 |
| 2 | rHA-Infestin-4 4 mg/kg | 6/11 (55 %) | 11 |
| 3 | C1-INH (BERINERT®) 20 IU/kg | 9/11 (82 %) | 11 |
| 4 | rHA-Infestin-4 (4 mg/kg) + C1-INH (BERINERT®) (20 IU/kg) | 2/10 (20 %) | 10 |

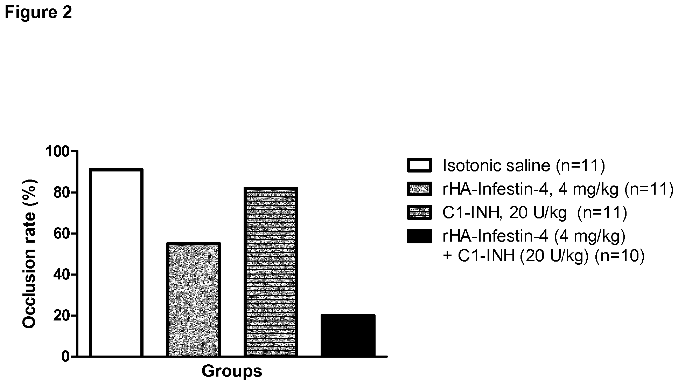

As shown in Table 4, the three minute ferric chloride lesion of the carotid artery resulted in thrombotic occlusion in 10/11 (91 %) isotonic saline-treated rats (Table 4). These vessel occlusions occurred within 32 minutes (Fig. 1). Pretreatment with 4 mg/kg rHA-Infestin-4 resulted in a partial prevention (55 % occlusion) of arterial thrombosis in a group of 11 rats. A dose of 20 IU/kg C1-INH (BERINERT®) was inefficient (i.e. 82 % occlusion), whereas BERINERT® in combination with 4 mg/kg rHA-Infestin-4 led to a substantial protection from arterial thrombosis (20 % occlusion). Figure 2 graphically compares the percentage of vessels occluded (i.e., the occlusion rates) for each treatment group.

This study demonstrated that protection from arterial thrombosis could be synergistically enhanced, if rHA-Infestin-4 (4 mg/kg) was combined with 20 IU/kg C1-INH (BERINERT®).

### Example 2: Murine EAE model of Multiple Sclerosis (out of scope)

The effect of combination treatment using at least one Factor XII inhibitor and at least one C1-INH is evaluated in a murine experimental autoimmune encephalomyelitis (EAE) animal model. (Stüve et al., Arch Neurol. 2010; 67:1307-15.) Treatment groups include a saline control group, a group treated with at least one Factor XII inhibitor, a group treated with at least one C1-INH, and a group treated with at least one Factor XII inhibitor and at least one C1-INH, optionally compared to groups treated with one or more corticosteroid, interferon, or glatiramer acetate. The C1-INH is BERINERT® (CSL Behring), and the FXII inhibitor is rHA-Infestin-4 or the anti-FXIIa antibody 3F7.

EAE is induced by immunization of 10-12 weeks old female C57BI/6 mice with 200 µg MOG₃₅₋₅₅. The C57BI/6 MOG₃₅₋₅₅ mouse model is commonly used for pre-clinical validation of therapeutic compounds. (Krishnamoorthy & Wekerle, Eur. J. Immunol 2009; 39:2031-35.) The course of EAE in this model resembles a primary progressive form of MS, since once induced the disease does not remit. Complete Freund's Adjuvant (CFA) is supplemented with MOG to obtain a 1 mg/ml emulsion and 2 x 100 µl are injected subcutaneously at two different sites of the flank of deeply anesthetized mice.

Treatment is administered by daily intravenous injection over a 50 day period. The course of EAE in the different treatment groups is monitored daily by two blinded investigators using the following score system: grade 0, no abnormality; grade 1, limp tail tip; grade 2, limp tail; grade 3, moderate hind limb weakness; grade 4, complete hind limb weakness; grade 5, mild paraparesis; grade 6, paraparesis; grade 7, heavy paraparesis or paraplegia; grade 8, tetraparesis; grade 9, quadriplegia or premoribund state; grade 10, death. Scores over the 50 day monitoring period are compared, looking for any alteration, additive, or synergistic effects across the different treatment groups. After 50 days, mice are sacrificed, tissue samples are collected, and evaluated histopathologically for EAE progression.

### Example 3: Rat Model of Skeletal Ischemia (out of scope)

The effect of combination treatment using at least one Factor XII inhibitor and at least one C1-INH is evaluated in a rat model of skeletal ischemia. Treatment groups include a saline control group, a group treated with at least one Factor XII inhibitor, a group treated with at least one C1-INH, and a group treated with at least one Factor XII inhibitor and at least one C1-INH. The C1-INH is BERINERT® (CSL Behring) and the FXII inhibitor is rHA-Infestin-4.

Male Wistar rats are anesthetized and limb fur is completely removed from both hind limbs with an electric shaver. The rats are kept on a heating pad to maintain body temperature at 37°C. Approximately, thirty minutes after the induction of anesthesia, unilateral hind limb ischemia is induced for 3 hours by clamping the femoral artery. After 3 hours of ischemia, the limb is reperfused for 24 hours.

Immunofluorescence is used to quantify the deposition of various components of the complement system as well as Fibrin/ Fibrinogen, Heparan Sulfate, Bradykinin receptor B₁ and Bradykinin receptor B₂ in tissue samples from the occluded and reperfused muscle. Tissue samples are washed in PBS, blotted dry, and embedded in matrix (OCT, Tissue tek) on dry ice. Samples are incubated with primary antibodies over night at 4°C, followed by secondary antibody incubation for 1 hour at room temperature. Immunofluorescence levels are quantified in the different samples, looking for any alteration, additive, or synergistic effects across the different treatment groups. Additionally, a tissue wet/dry ratio is calculated from tissue samples to evaluate effects on edema formation during skeletal IRI in an MTT (3-(4,5-dimethylthiazol-2-yl)-2,5-diphenyltetrazolium bromide, Sigma, St. Louis, USA) assay used to evaluate effects on muscle viability.

### Example 4: Mouse Model of Hereditary Angioedema

The effect of combination treatment using at least one Factor XII inhibitor and at least one C1-INH is evaluated in a mouse model of hereditary angioedema (HAE). Treatment groups include a saline control group, a group treated with at least one Factor XII inhibitor, a group treated with at least one C1-INH, and a group treated with at least one Factor XII inhibitor and at least one C1-INH, optionally compared to a group treated with a Kunitz domain plasma kallikrein inhibitor (DX88). The C1-INH is BERINERT® (CSL Behring), and the FXII inhibitor is rHA-Infestin-4 or the anti-FXIIa antibody 3F7.

Mice in which physiological C1-INH is not expressed (Han et al., J. Clin. Invest. 109:1057-1063 (2002)) are obtained and tested for symptoms of HAE. Mice are administered treatment, followed by injection with Evans blue dye (30 mg/kg in 100 µl PBS; Sigma Chemical Co., St. Louis, Missouri, USA) into the tail vein after dosing. Tissue samples are taken 30 minutes after injection with dye. Vascular permeability is measured by quantifying the degree of extravasation of dye from the vasculature, looking for any alteration, additive, or synergistic effects across the different treatment groups. Alternatively, other C1-INH-deficient mice (Oschatz et al., Immunity 34:258-268 (2011)) can be used for efficacy studies. Furthermore, after treatment with study drugs, different edema-provoking stimuli can be applied either locally or intravenously, followed by evaluation of edema formation or increased vascular permeability monitored via an intravenous tracer application. The preceding examples are intended to illustrate and in no way limit the present disclosure. Other embodiments of the disclosed compositions and methods will be apparent to those skilled in the art from consideration of the specification and practice of the compositions and methods disclosed herein.

## Claims

1. A composition comprising an effective amount of at least one Factor XII (FXII) inhibitor and at least one C1-Inhibitor (C1-INH) for the use of treating a disorder of the contact activation system selected from a thrombotic disorder and a disorder related to kinin formation,
wherein disorder related to kinin formation is selected from hereditary angioedema (HAE), secondary brain edema, edema of the central nervous system, hypotensive shock, and edema during or after contacting blood with an artificial surface,
wherein the at least one C1-INH is a plasma-derived human C1 Esterase Inhibitor and/or a recombinant human C1 Esterase Inhibitor,
wherein the FXII inhibitor is not C1 INH, and
wherein the at least one FXII inhibitor comprises
(i) the wild type Infestin-4 polypeptide sequence (SEQ ID NO: 1), or a polypeptide sequence comprising:
(a) SEQ ID NO: 1 modified to contain 1-5 amino acid mutations outside of N-terminal amino acid positions 2-13 of SEQ ID NO: 1;
and/or
(b) a homology of at least 70%, 80%, 85%, 90%, 95%, 98%, or 99% to SEQ ID NO: 1 and retaining six conserved cysteine residues from SEQ ID NO: 1;
and/or
(ii) an anti-FXII antibody wherein the anti-FXII antibody binds to and inhibits FXIIa activation and/or activity.

2. A composition for use according to claim 1, wherein the disorder is a thrombotic disorder and is a venous, arterial or capillary thrombus, a thrombus in the heart, a chronic or acute thromboembolism, thrombus formation during or after contacting blood with an artificial surface, a spinal cord injury, a traumatic brain injury, a secondary brain edema, or an edema of the central nervous system.

3. A composition for use according to claim 2, wherein the thrombotic disorder is a venous, arterial, or capillary thrombus, and is a stroke, myocardial infarction, deep vein thrombosis (DVT), portal vein thrombosis, thromboembolism, renal vein thrombosis, jugular vein thrombosis, cerebral venous sinus thrombosis, Budd-Chiari syndrome, Paget-Schroetter disease, or silent brain ischemia.

4. A composition for use according to any one of the claims 1 to 3, wherein the anti-FXII antibody comprises
(i)
(a) a VH region comprising heavy chain CDR1 as set forth in SEQ ID NO: 8, heavy chain CDR2 as set forth in SEQ ID NO: 10, and heavy chain CDR3 as set forth in SEQ ID NO: 12;
and/or
(b) a VL region comprising light chain CDR1 as set forth in SEQ ID NO: 13, light chain CDR2 as set forth in SEQ ID NO: 14, and light chain CDR3 as set forth in SEQ ID NO: 16.;
or
(ii)
(a) a VH region comprising heavy chain CDR1 as set forth in SEQ ID NO: 8, heavy chain CDR2 as set forth in SEQ ID NO: 9, and heavy chain CDR3 as set forth in SEQ ID NO: 11;
and/or
(b) a VL region comprising light chain CDR1 as set forth in SEQ ID NO: 13, light chain CDR2 as set forth in SEQ ID NO: 14, and light chain CDR3 as set forth in SEQ ID NO: 15;
or
(iii) a VH region comprising SEQ ID NO: 6 and a VL region comprising SEQ ID NO: 7.

5. A composition for use according to any one of claims 1-4, wherein the anti-FXII antibody is an IgG antibody.

6. A composition for use according to any one of claims 1-3, wherein the at least one FXII inhibitor is linked to a fusion partner comprising PEG or a half-life enhancing polypeptide selected from albumin, afamin, alpha-fetoprotein, vitamin D binding protein, human albumin, an immunoglobulin, and an Fc of an IgG.

7. A composition for use according to claim 6, wherein the half-life enhancing polypeptide is linked to the FXII inhibitor via a linker.

8. A composition for use according to any one of claims 6-7, wherein the at least one FXII inhibitor is a fusion protein comprising human albumin joined to a FXII inhibitor via a linker peptide.

9. A composition for use according to any one of claims 1-8, wherein the at least one FXII inhibitor and the at least one C1-INH are administered after a patient develops the disorder of the contact activation system.

10. A composition for use according to any one of the claims 1-9, wherein the at least one FXII inhibitor and the at least one C1-INH are administered (i) in a single dose as an injection or an infusion, (ii) in multiple doses, each as an injection or an infusion, or (iii) as a continuous infusion or application.

11. A composition for use according to claim 10, wherein the at least one FXII inhibitor and the at least one C1-INH are administered intravenously.

12. A composition for use according to any one of claims 1-11, wherein the at least one FXII inhibitor and the at least one C1-INH are administered at the same time or wherein the at least one FXII inhibitor and the at least one C1-INH are administered sequentially, with either the FXII inhibitor administered first or the C1-INH administered first.

13. A composition for use according to claim 12, wherein the at least one C1-INH is administered immediately after the at least one FXII inhibitor, or up to about 10 minutes after the at least one FXII inhibitor
or wherein the at least one FXII inhibitor is administered immediately after the at least one C1-INH or up to about 10 minutes after the at least one C1-INH.

14. A composition for use according to any one of the claims 1-13, wherein the at least one FXII inhibitor is administered at a concentration ranging from about 0.01 to about 1000 mg/kg body weight, or about 1 to about 500 mg/kg; and/or the at least one C1-INH is administered at a concentration ranging from about 0.01 IU/kg to about 5000 IU/kg of bodyweight, or about 5 to about 500 IU/kg.

15. A composition for use of any one of claims 1-14, wherein the at least one FXII inhibitor and the at least one C1-INH are administered immediately after, or up to 1 hour, six hours, 12 hours, 1 day, 3 days, 5 days, or 10 days after an initial insult or episode of the disorder of the contact activation system.

16. A composition for use according to claim 15, wherein the at least one FXII inhibitor and the at least one C1-INH are administered at least twice, at least three times, or at least five times.

## Patentansprüche

1. Zusammensetzung, umfassend eine wirksame Menge wenigstens eines Faktor-XII(FXII)-Inhibitors und wenigstens eines C1-Inhibitors (C1-INH), zur Verwendung beim Behandeln einer Störung des Kontaktaktivierungssystem ausgewählt aus einer thrombotischen Störung und einer Störung in Verbindung mit Kininbildung,
wobei die Störung in Verbindung mit Kininbildung ausgewählt ist aus hereditärem Angioödem (HAE), sekundärem Hirnödem, Ödem des Zentralnervensystems, hypotonischem Schock und Ödem während oder nach In-Kontakt-Bringen von Blut mit einer künstlichen Oberfläche,
wobei es sich bei dem wenigstens einen C1-INH um einen aus Plasma stammenden menschlichen C1-Esterase-Inhibitor und/oder einen rekombinanten menschlichen C1-Esterase-Inhibitor handelt,
wobei es sich bei dem FXII-Inhibitor nicht um C1-INH handelt und
wobei der wenigstens eine FXII-Inhibitor Folgendes umfasst:
(i) die Wildtyp-Infestin-4-Polypeptidsequenz (SEQ ID NO: 1) oder eine Polypeptidsequenz, die Folgendes umfasst:
(a) SEQ ID NO: 1, modifiziert, so dass 1-5 Aminosäuremutationen außerhalb der N-terminalen Aminosäurepositionen 2-13 von SEQ ID NO: 1 enthalten sind;
und/oder
(b) eine Homologie von wenigstens 70%, 80%, 85%, 90%, 95%, 98% oder 99% zu SEQ ID NO: 1 unter Beibehaltung sechs konservierter Cysteinreste aus SEQ ID NO: 1;
und/oder
(ii) einen Anti-FXII-Antikörper, wobei der Anti-FXII-Antikörper an FXIIa-Aktivierung und/oder -Aktivität bindet und diese hemmt.

2. Zusammensetzung zur Verwendung nach Anspruch 1, wobei es sich bei der Störung um eine thrombotische Störung und zwar eine Venen-, Arterien- oder Kapillarthrombose, eine Thrombose im Herz, eine chronische oder akute Thromboembolie, Thrombosebildung während oder nach In-Kontakt-Bringen von Blut mit einer künstlichen Oberfläche, eine Rückenmarkverletzung, ein Hirntrauma, ein sekundäres Hirnödem oder ein Ödem des Zentralnervensystems handelt.

3. Zusammensetzung zur Verwendung nach Anspruch 2, wobei es sich bei der thrombotischen Störung um eine Venen-, Arterien- oder Kapillarthrombose und zwar einen Schlaganfall, Myokardinfarkt, eine tiefe Venenthrombose (Deep Vein Thrombosis, DVT), Pfortaderthrombose, Thromboembolie, Nierenvenenthrombosis, Halsvenenthrombose, zerebrale Venen- und Sinusthrombose, Budd-Chiari-Syndrom, Paget-Schroetter-Syndrom oder stille Hirnischämie handelt.

4. Zusammensetzung zur Verwendung nach einem der Ansprüche 1 bis 3, wobei der Anti-FXII-Antikörper Folgendes umfasst:
(i)
(a) eine VH-Region umfassend die Schwere-Kette-CDR1 gemäß SEQ ID NO: 8, Schwere-Kette-CDR2 gemäß SEQ ID NO: 10 und Schwere-Kette-CDR3 gemäß SEQ ID NO: 12;
und/oder
(b) eine VL-Region umfassend die Leichte-Kette-CDR1 gemäß SEQ ID NO: 13, Leichte-Kette-CDR2 gemäß SEQ ID NO: 14 und Leichte-Kette-CDR3 gemäß SEQ ID NO: 16;
oder
(ii)
(a) eine VH-Region umfassend die Schwere-Kette-CDR1 gemäß SEQ ID NO: 8, Schwere-Kette-CDR2 gemäß SEQ ID NO: 9 und Schwere-Kette-CDR3 gemäß SEQ ID NO: 11;
und/oder
(b) eine VL-Region umfassend die Leichte-Kette-CDR1 gemäß SEQ ID NO: 13, Leichte-Kette-CDR2 gemäß SEQ ID NO: 14 und Leichte-Kette-CDR3 gemäß SEQ ID NO: 15;
oder
(iii) eine VH-Region umfassend die SEQ ID NO: 6, und eine VL-Region umfassend die SEQ ID NO: 7.

5. Zusammensetzung zur Verwendung nach einem der Ansprüche 1-4, wobei es sich bei dem Anti-FXII-Antikörper um einen IgG-Antikörper handelt.

6. Zusammensetzung zur Verwendung nach einem der Ansprüche 1-3, wobei der wenigstens eine FXII-Inhibitor mit einem Fusionspartner verknüpft ist, der PEG oder ein halbwertszeitverlängerndes Polypeptid ausgewählt aus Albumin, Afamin, Alpha-Fetoprotein, Vitamin-D-Bindungsprotein, Humanalbumin, ein Immunglobulin und ein Fc eines IgG umfasst.

7. Zusammensetzung zur Verwendung nach Anspruch 6, wobei das halbwertszeitverlängernde Polypeptid mit dem FXII-Inhibitor über einen Linker verknüpft ist.

8. Zusammensetzung zur Verwendung nach einem der Ansprüche 6-7, wobei es sich bei dem wenigstens einen FXII-Inhibitor um ein Fusionsprotein handelt, das Humanalbumin verbunden über ein Linkerpeptid mit einem FXII-Inhibitor umfasst.

9. Zusammensetzung zur Verwendung nach einem der Ansprüche 1-8, wobei der wenigstens eine FXII-Inhibitor und der wenigstens eine C1-INH verabreicht werden, nachdem sich bei einem Patienten die Störung des Kontaktaktivierungssystems entwickelt hat.

10. Zusammensetzung zur Verwendung nach einem der Ansprüche 1-9, wobei der wenigstens eine FXII-Inhibitor und derwenigstens eine C1-INH (i) in einer Einmaldosis als Injektion oder Infusion, (ii) in mehreren Dosen, jeweils als Injektion oder Infusion, oder (iii) als Dauerinfusion oder -anwendung verabreicht werden.

11. Zusammensetzung zur Verwendung nach Anspruch 10, wobei der wenigstens eine FXII-Inhibitor und der wenigstens eine C1-INH intravenös verabreicht werden.

12. Zusammensetzung zur Verwendung nach einem der Ansprüche 1-11, wobei der wenigstens eine FXII-Inhibitor und der wenigstens eine C1-INH gleichzeitig verabreicht werden
oder wobei der wenigstens eine FXII-Inhibitor und der wenigstens eine C1-INH nacheinander verabreicht werden, wobei entweder der FXII-Inhibitor oder der C1-INH zuerst verabreicht wird.

13. Zusammensetzung zur Verwendung nach Anspruch 12, wobei der wenigstens eine C1-INH unmittelbar nach dem wenigstens einen FXII-Inhibitor oder bis zu etwa 10 Minuten nach dem wenigstens einen FXII-Inhibitor verabreicht wird
oder wobei derwenigstens eine FXII-Inhibitor unmittelbar nach dem wenigstens einen C1-INH oder bis zu etwa 10 Minuten nach dem wenigstens einen C1-INH verabreicht wird.

14. Zusammensetzung zur Verwendung nach einem der Ansprüche 1-13, wobei der wenigstens eine FXII-Inhibitor in einer Konzentration im Bereich von etwa 0,01 bis etwa 1000 mg/kg Körpergewicht oder etwa 1 bis etwa 500 mg/kg verabreicht wird; und/oder der wenigstens eine C1-INH in einer Konzentration im Bereich von etwa 0,01 IU/kg bis etwa 5000 IU/kg Körpergewicht oder etwa 5 bis etwa 500 IU/kg verabreicht wird.

15. Zusammensetzung zur Verwendung gemäß einem der Ansprüche 1-14, wobei der wenigstens eine FXII-Inhibitor und der wenigstens eine C1-INH unmittelbar nach oder bis zu 1 Stunde, sechs Stunden, 12 Stunden, 1 Tag, 3 Tagen, 5 Tagen oder 10 Tagen nach einer ersten Attacke oder Episode der Störung des Kontaktaktivierungssystems verabreicht werden.

16. Zusammensetzung zur Verwendung nach Anspruch 15, wobei der wenigstens eine FXII-Inhibitor und der wenigstens eine C1-INH wenigstens zweimal, wenigstens dreimal oder wenigstens fünfmal verabreicht werden.

## Revendications

1. Composition comprenant une quantité efficace d'au moins un inhibiteur du facteur XII (FXII) et au moins un inhibiteur de C1 (C1-INH) pour utilisation dans le traitement d'un trouble du système d'activation de contact choisi parmi un trouble thrombotique et un trouble lié à la formation de kinine,
où le trouble lié à la formation de kinine est choisi parmi un angioœdème héréditaire (AOH), un œdème cérébral secondaire, un œdème du système nerveux central, un choc hypotensif et un œdème pendant ou après mise en contact du sang avec une surface artificielle,
dans laquelle l'au moins un C1-INH est un inhibiteur d'estérase C1 humaine dérivée de plasma et/ou un inhibiteur d'estérase C1 humaine recombinante,
dans laquelle l'inhibiteur de FXII n'est pas C1-INH, et
dans laquelle l'au moins un inhibiteur de FXII comprend
(i) la séquence polypeptidique d'infestine 4 de type sauvage (SEQ ID NO: 1), ou une séquence polypeptidique comprenant :
(a) SEQ ID NO : 1 modifié pour contenir 1 à 5 mutations d'acide aminé à l'extérieur des positions d'acide aminé N-terminales 2 à 13 de SEQ ID NO: 1 ;
et/ou
(b) une homologie d'au moins 70 %, 80 %, 85 %, 90 %, 95 %, 98 %, ou 99 % avec SEQ ID NO : 1 et conservant six résidus cystéine conservés de SEQ ID NO: 1 ;
et/ou
(ii) un anticorps anti-FXII où l'anticorps anti-FXII se lie à, et inhibe, l'activation et/ou l'activité de FXIIa.

2. Composition pour utilisation selon la revendication 1, où le trouble est un trouble thrombotique et un thrombus veineux, artériel ou capillaire, un thrombus dans le cœur, une maladie thromboembolique chronique ou aiguë, une formation de thrombus pendant ou après contact du sang avec une surface artificielle, une lésion de la moelle épinière, un traumatisme crânien, un œdème cérébral secondaire ou un œdème du système nerveux central.

3. Composition pour utilisation selon la revendication 2, où le trouble thrombotique est un thrombus veineux, artériel ou capillaire, et est un accident vasculaire cérébral, un infarctus du myocarde, une thrombose veineuse profonde (TVP), une thrombose de la veine porte, une maladie thromboembolique, une thrombose de la veine rénale, une thrombose de la veine jugulaire, une thrombose des sinus veineux cérébraux, le syndrome de Budd-Chiari, la maladie de Paget-Schroetter ou une ischémie cérébrale silencieuse.

4. Composition pour utilisation selon l'une quelconque des revendications 1 à 3, dans laquelle l'anticorps anti-FXII comprend
(i)
(a) une région VH comprenant une CDR1 de chaîne lourde telle que décrite dans SEQ ID NO : 8, une CDR2 de chaîne lourde telle que décrite dans SEQ ID NO: 10, et une CDR3 de chaîne lourde telle que décrite dans SEQ ID NO: 12;
et/ou
(b) une région VL comprenant une CDR1 de chaîne légère telle que décrite dans SEQ ID NO : 13, une CDR2 de chaîne légère telle que décrite dans SEQ ID NO: 14, et une CDR3 de chaîne légère telle que décrite dans SEQ ID NO: 16;
ou
(ii)
(a) une région VH comprenant une CDR1 de chaîne lourde telle que décrite dans SEQ ID NO: 8, une CDR2 de chaîne lourde telle que décrite dans SEQ ID NO: 9, et une CDR3 de chaîne lourde telle que décrite dans SEQ ID NO: 11;
et/ou
(b) une région VL comprenant une CDR1 de chaîne légère telle que décrite dans SEQ ID NO: 13, une CDR2 de chaîne légère telle que décrite dans SEQ ID NO: 14, et une CDR3 de chaîne légère telle que décrite dans SEQ ID NO: 15;
ou
(iii) une région VH comprenant SEQ ID NO: 6 et une région VL comprenant SEQ ID NO: 7.

5. Composition pour utilisation selon l'une quelconque des revendications 1 à 4, dans laquelle l'anticorps anti-FXII est un anticorps IgG.

6. Composition pour utilisation selon l'une quelconque des revendications 1 à 3, dans laquelle l'au moins un inhibiteur de FXII est lié à un partenaire de fusion comprenant PEG ou un polypeptide augmentant la demi-vie choisi parmi l'albumine, l'afamine, l'alpha-fétoprotéine, la protéine de liaison de la vitamine D, l'albumine humaine, une immunoglobuline, et un Fc d'une IgG.

7. Composition pour utilisation selon la revendication 6, dans laquelle le polypeptide augmentant la demi-vie est lié à l'inhibiteur de FXII par l'intermédiaire d'un lieur.

8. Composition pour utilisation selon l'une quelconque des revendications 6 à 7, dans laquelle l'au moins un inhibiteur de FXII est une protéine de fusion comprenant l'albumine humaine assemblée à un inhibiteur de FXII par l'intermédiaire d'un lieur peptidique.

9. Composition pour utilisation selon l'une quelconque des revendications 1 à 8, dans laquelle l'au moins un inhibiteur de FXII et l'au moins un C1-INH sont administrés une fois qu'un patient a développé le trouble du système d'activation de contact.

10. Composition pour utilisation selon l'une quelconque des revendications 1 à 9, dans laquelle l'au moins un inhibiteur de FXII et l'au moins un C1-INH sont administrés (i) en dose unique sous la forme d'une injection ou d'une perfusion, (ii) en doses multiples, chacune sous la forme d'une injection ou d'une perfusion, ou (iii) sous la forme d'une perfusion ou application continue.

11. Composition pour utilisation selon la revendication 10, dans laquelle l'au moins un inhibiteur de FXII et l'au moins un C1-INH sont administrés par voie intraveineuse.

12. Composition pour utilisation selon l'une quelconque des revendications 1 à 11, dans laquelle l'au moins un inhibiteur de FXII et l'au moins un C1-INH sont administrés simultanément
ou dans laquelle l'au moins un inhibiteur de FXII et l'au moins un C1-INH sont administrés séquentiellement, avec l'inhibiteur de FXII administré en premier ou le C1-INH administré en premier.

13. Composition pour utilisation selon la revendication 12, dans laquelle l'au moins un C1-INH est administré immédiatement après l'au moins un inhibiteur de FXII, ou jusqu'à environ 10 minutes après l'au moins un inhibiteur de FXII
ou dans laquelle l'au moins un inhibiteur de FXII est administré immédiatement après l'au moins un C1-INH ou jusqu'à environ 10 minutes après l'au moins un C1-INH.

14. Composition pour utilisation selon l'une quelconque des revendications 1 à 13, dans laquelle l'au moins un inhibiteur de FXII est administré à une concentration dans la plage d'environ 0,01 à environ 1000 mg/kg de poids corporel, ou environ 1 à environ 500 mg/kg ; et/ou l'au moins un C1-INH est administré à une concentration dans la plage d'environ 0,01 UI/kg à environ 5000 UI/kg de poids corporel, ou environ 5 à environ 500 UI/kg.

15. Composition pour utilisation selon l'une quelconque des revendications 1 à 14, dans laquelle l'au moins un inhibiteur de FXII et l'au moins un C1-INH sont administrés immédiatement après, ou jusqu'à 1 heure, six heures, 12 heures, 1 jour, 3 jours, 5 jours, ou 10 jours après une atteinte initiale ou un épisode du trouble du système d'activation de contact.

16. Composition pour utilisation selon la revendication 15, dans laquelle l'au moins un inhibiteur de FXII et l'au moins un C1-INH sont administrés au moins deux fois, au moins trois fois, ou au moins cinq fois.


### SEQUENCE LISTING

<110> CSL Behring GmbH
<120> Combination Therapy using a Factor XII Inhibitor and a C1-Inhibitor
<130> 2013_M003_A188
<150> US61/840662
   <151> 2013-06-28
<150> EP 13176605.7
   <151> 2013-07-16
<160> 21
<170> PatentIn version 3.5
<210> 1
   <211> 48
   <212> PRT
   <213> Triatoma infestans
<400> 1
   Glu Val Arg Asn Pro Cys Ala Cys Phe Arg Asn Tyr Val Pro Val Cys
   1               5                   10                  15
   Gly Ser Asp Gly Lys Thr Tyr Gly Asn Pro Cys Met Leu Asn Cys Ala
               20                  25                  30
   Ala Gln Thr Lys Val Pro Gly Leu Lys Leu Val His Glu Gly Arg Cys
           35                  40                  45
<210> 2
   <211> 56
   <212> PRT
   <213> Homo sapiens
<400> 2
   Asp Ser Leu Gly Arg Glu Ala Lys Cys Tyr Asn Glu Leu Asn Gly Cys
   1               5                   10                  15
   Thr Lys Ile Tyr Asp Pro Val Cys Gly Thr Asp Gly Asn Thr Tyr Pro
               20                  25                  30
   Asn Glu Cys Val Leu Cys Phe Glu Asn Arg Lys Arg Gln Thr Ser Ile
           35                  40                  45
   Leu Ile Gln Lys Ser Gly Pro Cys
       50                  55
<210> 3
   <211> 53
   <212> PRT
   <213> Homo sapiens
<400> 3
   Asp Ser Leu Gly Arg Glu Val Arg Asn Pro Cys Ala Cys Phe Arg Asn
   1               5                   10                  15
   Tyr Val Pro Val Cys Gly Thr Asp Gly Asn Thr Tyr Pro Asn Glu Cys
               20                  25                  30
   Val Leu Cys Phe Glu Asn Arg Lys Arg Gln Thr Ser Ile Leu Ile Gln
           35                  40                  45
   Lys Ser Gly Pro Cys
       50
<210> 4
   <211> 53
   <212> PRT
   <213> Homo sapiens
<400> 4
   Asp Ser Leu Gly Arg Glu Val Arg Asn Pro Cys Ala Cys Phe Arg Asn
   1               5                   10                  15
   Tyr Val Pro Val Cys Gly Thr Asp Gly Asn Thr Tyr Gly Asn Glu Cys
               20                  25                  30
   Met Leu Cys Ala Glu Asn Arg Lys Arg Gln Thr Ser Ile Leu Ile Gln
           35                  40                  45
   Lys Glu Gly Pro Cys
       50
<210> 5
   <211> 54
   <212> PRT
   <213> Homo sapiens
<400> 5
   Asp Ser Leu Gly Arg Glu Val Arg Asn Pro Cys Ala Cys Phe Arg Asn
   1               5                   10                  15
   Tyr Val Pro Val Cys Gly Thr Asp Gly Asn Thr Tyr Gly Asn Glu Cys
               20                  25                  30
   Met Leu Asn Cys Ala Glu Asn Arg Lys Arg Gln Thr Ser Ile Leu Ile
           35                  40                  45
   Gln Lys Glu Gly Pro Cys
       50
<210> 6
   <211> 129
   <212> PRT
   <213> Homo sapiens
<400> 6
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Lys Tyr
   20 25 30
   Ile Met Gln Trp Val Arg Gln Ala Pro Gly Lys Gly Leu Glu Trp Val
   35 40 45
   Ser Gly Ile Arg Pro Ser Gly Gly Thr Thr Val Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Ala Leu Pro Arg Ser Gly Tyr Leu Ile Ser Pro His Tyr Tyr
   100 105 110
   Tyr Tyr Ala Leu Asp Val Trp Gly Gln Gly Thr Thr Val Thr Val Ser
   115 120 125
   Ser
<210> 7
   <211> 110
   <212> PRT
   <213> Homo sapiens
<400> 7
   Gln Ser Glu Leu Thr Gln Pro Pro Ser Ala Ser Gly Thr Pro Gly Gln
   1 5 10 15
   Arg Val Thr Ile Ser Cys Ser Gly Ser Ser Ser Asn Ile Gly Arg Asn
   20 25 30
   Tyr Val Tyr Trp Tyr Gln Gln Val Pro Gly Thr Ala Pro Lys Leu Leu
   35 40 45
   Ile Tyr Ser Asn Asn Gln Arg Pro Ser Gly Val Pro Asp Arg Phe Ser
   50 55 60
   Gly Ser Lys Ser Gly Thr Ser Ala Ser Leu Val Ile Ser Gly Leu Arg
   65 70 75 80
   Ser Glu Asp Glu Ala Asp Tyr Tyr Cys Ala Ala Trp Asp Ala Ser Leu
   85 90 95
   Arg Gly Val Phe Gly Gly Gly Thr Lys Leu Thr Val Leu Gly
   100 105 110
<210> 8
   <211> 5
   <212> PRT
   <213> Homo sapiens
<400> 8
   Lys Tyr Ile Met Gln
   1 5
<210> 9
   <211> 17
   <212> PRT
   <213> Homo sapiens
<400> 9
   Gly Ile Arg Pro Ser Gly Gly Thr Thr Val Tyr Ala Asp Ser Val Lys
   1 5 10 15
   Gly
<210> 10
   <211> 17
   <212> PRT
   <213> Artificial
<220>
   <223> Heavy chain CDR2 showing variations
<220>
   <221> variant
   <222> (3)..(3)
   <223> Xaa is selected from Arg, Asn, and Asp
<220>
   <221> variant
   <222> (4)..(4)
   <223> Xaa is selcted from Pro, Val, Ile and Met
<220>
   <221> variant
   <222> (5)..(5)
   <223> Xaa is selected from Ser, Pro and Ala
<220>
   <221> variant
   <222> (6)..(6)
   <223> Xaa is selected from Gly, Leu, Val and Thr
<220>
   <221> variant
   <222> (7)..(7)
   <223> Xaa is selected from Gly, Tyr, Gln, Lys, Arg, Asn and Met
<220>
   <221> variant
   <222> (8)..(8)
   <223> Xaa is selected from Thr, Gly and Ser
<400> 10
   Gly Ile Xaa Xaa Xaa Xaa Xaa Xaa Thr Val Tyr Ala Asp Ser Val Lys
   1               5                   10                  15
   Gly
<210> 11
   <211> 20
   <212> PRT
   <213> Homo sapiens
<400> 11
   Ala Leu Pro Arg Ser Gly Tyr Leu Ile Ser Pro His Tyr Tyr Tyr Tyr
   1               5                   10                  15
   Ala Leu Asp Val
               20
<210> 12
   <211> 20
   <212> PRT
   <213> Artificial
<220>
   <223> Heavy chain CDR3 showing variations
<220>
   <221> variant
   <222> (9)..(9)
   <223> Xaa is selected from Ala, Met and Val
<220>
   <221> variant
   <222> (10)..(10)
   <223> Xaa is selcted from Ser and Lys
<220>
   <221> variant
   <222> (11)..(11)
   <223> Xaa is selected from Pro, Lys, Thr and His
<220>
   <221> variant
   <222> (12)..(12)
   <223> Xaa is selected from His, Asn, Gly and Gln
<400> 12
   Ala Leu Pro Arg Ser Gly Tyr Leu Xaa Xaa Xaa Xaa Tyr Tyr Tyr Tyr
   1               5                   10                  15
   Ala Leu Asp Val
               20
<210> 13
   <211> 13
   <212> PRT
   <213> Homo sapiens
<400> 13
   Ser Gly Ser Ser Ser Asn Ile Gly Arg Asn Tyr Val Tyr
   1               5                   10
<210> 14
   <211> 7
   <212> PRT
   <213> Homo Sapiens
<400> 14
   Ser Asn Asn Gln Arg Pro Ser
   1               5
<210> 15
   <211> 10
   <212> PRT
   <213> Homo sapiens
<400> 15
   Ala Ala Trp Asp Ala Ser Leu Arg Gly Val
   1               5                   10
<210> 16
   <211> 10
   <212> PRT
   <213> Artificial
<220>
   <221> variant
   <222> (2)..(2)
   <223> Xaa is selected from Ala and Ser
<220>
   <221> variant
   <222> (4)..(6)
   <223> Xaa can be any amino acid
<220>
   <221> variant
   <222> (7).. (7)
   <223> Xaa is selected from Leu and Val
<220>
   <221> variant
   <222> (9)..(10)
   <223> Xaa can be any amino acid
<400> 16
   Ala Xaa Trp Xaa Xaa Xaa Xaa Arg Xaa Xaa
   1               5                   10
<210> 17
   <211> 13
   <212> PRT
   <213> Homo sapiens
<400> 17
   Ser Gly Ser Ser Glu Met Thr Val His His Tyr Val Tyr
   1               5                   10
<210> 18
<400> 18
   000
<210> 19
   <211> 585
   <212> PRT
   <213> Homo sapiens
<400> 19
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Lys Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
               580                 585
<210> 20
   <211> 12
   <212> PRT
   <213> Triatoma infestans
<400> 20
   Val Arg Asn Pro Cys Ala Cys Phe Arg Asn Tyr Val
   1               5                   10
<210> 21
   <211> 12
   <212> PRT
   <213> Triatoma infestans
<400> 21
   Asp Ser Leu Gly Arg Glu Val Arg Asn Pro Cys Ala
   1               5                   10